# EUROPEAN PATENT APPLICATION

(11) **EP 4 707 395 A1**
(43) Date of publication of application: **11.03.2026**
(21) Application number: 24803490.2
(22) Date of filing: 08.05.2024
(51) Int. Cl.: C12N 15/62, C07K 14/37, C07K 19/00, C12N 15/31

(54) **PHOTOSWITCH PROTEIN**

(30) Priority: 10.05.2023 JP 2023077641
(71) Applicant: The University of Tokyo, Bunkyo-ku, Tokyo 113-8654 (JP); KANAGAWA INSTITUTE OF INDUSTRIAL SCIENCE AND TECHNOLOGY, Ebina-shi Kanagawa 243-0435 (JP)
(72) Inventor: SATO, Moritoshi, Tokyo 113-8654 (JP); KAWANO, Fuun, Tokyo 113-8654 (JP); OTABE, Takahiro, Ebina-shi, Kanagawa 243-0435 (JP)
(74) Representative: Isarpatent
(86) International application number: PCT/JP2024/017063
(87) International publication number: WO 2024/232381

(57) **Abstract**

It is an object of the present invention to provide a photoswitching protein that can control the binding and dissociation of proteins more efficiently than conventional photoswitching proteins.

It is a photoswitching protein consisting of a single polypeptide, differing from the conventional photoswitching protein consisting of two different proteins. More specifically, the photoswitching protein of the present invention is, for example, a protein in which the C-terminus of the following protein (a) is directly or indirectly linked to the N-terminus of the following protein (b):
(a) a protein comprising an amino acid sequence having a sequence identity of 80% or more to an amino acid sequence comprising a deletion of an X number of consecutive amino acid residues from the C-terminal residue to the N-terminal side of the amino acid sequence as set forth in SEQ ID No: 1 or SEQ ID No: 36, wherein X represents an integer of 0 or greater and 6 or smaller; and
(b) a protein comprising an amino acid sequence having a sequence identity of 80% or more to an amino acid sequence comprising a deletion of a Y number of consecutive amino acid residues from the N-terminal residue to the C-terminal side of the amino acid sequence as set forth in SEQ ID No: 1 or SEQ ID No: 36, wherein Y represents an integer of 0 or greater and 55 or smaller.

## Description

### Technical Field

The present invention relates to a photoreceptor protein (photoswitching protein) that can control the binding and dissociation of interacting proteins.

### Background Art

Protein, which is one of biological materials, plays a various functions in a living body. For example, proteins are involved in the control of systems that are fundamental to life, such as intracellular signal transduction and material transport, through interactions between the proteins. In recent years, with the development of optogenetic techniques (optogenetics), techniques have been developed to control interactions between proteins in a living body by light manipulation, and for example, photoswitching proteins are currently attracting attention. Photoswitching proteins have initially been reported as a pair of proteins whose binding and dissociation can be controlled by turning light irradiation ON and OFF (Patent Literature 1). Through the binding and dissociation of such photoswitching proteins by light manipulation, various intracellular phenomena, such as signal transduction, genome editing and gene expression, can be controlled (Non Patent Literature 1 and Non Patent Literature 2).

Vivid (VVD), a protein derived from Neurospora crassa, receives a blue light and rapidly forms a homodimer (Non Patent Literature 3). VVD, consisting of approximately 150 amino acids, has a smaller molecular weight than previously reported photoreceptor-based dimeric proteins, and facilitates precise molecular design. Moreover, since the cofactor (flavin adenine dinucleotide) required for VVD activity is present in eukaryotic cells, VVD is advantageous in that it is excellent in usability for functioning in mammalian cells. However, VVD has been problematic in that it has low binding affinity upon light irradiation, and in that it cannot select binding partners because it forms a homodimer.

In order to address the above-mentioned problems of VVD, the present inventors have discovered that modifying the amino acids in the α-helix on the N-terminal side of VVD enables the control of the dimerization efficiency, and modifying the amino acids adjacent to the flavin-binding domain enables the control of the dimer dissociation rate. Furthermore, the present inventors have also revealed that, with regard to such amino acid modifications of VVD, by adding different modifications to each of the dimer-forming pairs, a dimer is formed between VVD proteins with different modifications (Patent Literature 1 and Non Patent Literature 1). Hereinafter, these VVD proteins with different amino acid modifications will be also referred to as "Magnets," and the protein control system using the Magnets will be also referred to as a "Magnet system."

The Magnet system developed by the present inventors is composed of a VVD in which at least one of the amino acids located in the contact region during dimer formation has been substituted with a basic amino acid (an amino acid having a positive charge) (wherein this modified VVD is also referred to as a "positive magnet (pMag)"), and a VVD in which at least one of the amino acids located in the contact region during dimer formation has been substituted with an acidic amino acid (an amino acid having a negative charge) (wherein this modified VVD is also referred to as a "negative magnet (nMag)"). The pair of pMag and nMag is expected to be utilized as a photoswitching protein that overcomes the above-mentioned problems of wild-type VVD. However, the binding efficiency between interacting proteins by two independent molecules, pMag and nMag, depends on the frequency of molecular interaction, and when a two-molecule-type Magnet system is used in cells, it has depended on the intracellular concentration of the Magnet protein. In other words, although the Magnet system shows improved control efficiency, there still has been room for further improvement in terms of increasing the interaction frequency.

### Citation List

### Patent Literature

Patent Literature 1: JP Patent Publication (Kokai) No. 2015-165776 A

### Non Patent Literature

Non Patent Literature 1: Kawano et al., Nature Chemical Biology 12: 1059-1064 (2016).
Non Patent Literature 2: Kawano et al., Nature Communications 6: 6256 (2015).
Non Patent Literature 3: Zoltowski et al., Science 316: 1054-1057 (2007).
Non Patent Literature 4: Nihongaki et al., Nat. Biotechnol. 33: 755-760 (2015).

### Summary of Invention

### Technical Problem

In view of the above-described circumstances, it is an object of the present invention to provide a photoswitching protein that can control the binding and dissociation of proteins more efficiently than conventional photoswitching proteins (for example, Magnets, etc.).

### Solution to Problem

In order to solve the above-mentioned problems, the present inventors have prepared a one-molecule-type photoswitching protein by linking pMag to nMag in series, which constitute the aforementioned Magnets. As a result, the inventors have found that the prepared photoswitching protein dramatically improves the efficiency of controlling the binding and dissociation of split proteins (protein fragments prepared by splitting an originally single protein such that they can reassemble to the single protein), compared to a previous two-molecule-type photoswitching protein. Hereinafter, a one-molecule-type photoswitching protein by linking VVD proteins or mutants thereof (pMag, nMag, etc.) in series is also referred to as an "scMagnet."

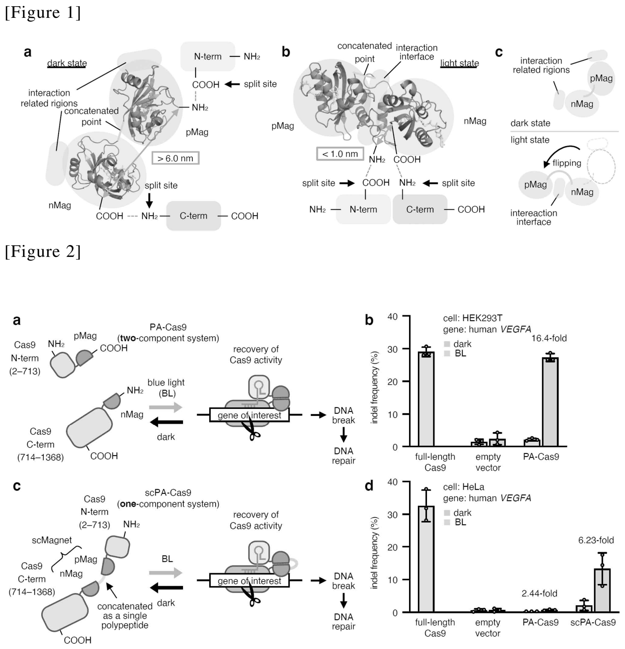

The present inventors have designed the scMagnet based on the three-dimensional structural models of pMag and nMag. When pMag and nMag are directly linked to each other, the N-terminal fragment and the C-terminal fragment of each split protein binding to pMag and nMag are estimated to be at least 6 nm apart from each other (Figure 1a), assuming from the structural models of pMag and nMag in the dark. Therefore, self-rebinding of split proteins in the dark is considered to be suppressed by aligning and linking pMag and nMag in series (Figure 1a). On the other hand, assuming from the structural model of a VVD dimer (a dimer consisting of pMag and nMag) under light irradiation, the N-terminus of pMag and the C-terminus of nMag are estimated to be closely located within a range of 1 nm of each other, which is thought to promote the rebinding of the split proteins (Figure 1b).

Based on these considerations, the present inventors have hypothesized that scMagnet undergoes a significant light-dependent structural change from an open conformation to a closed conformation, and that the binding and dissociation of individual proteins (e.g., individual fragments of split proteins) binding to pMag and nMag can be extremely efficiently controlled by the intermolecular interaction between pMag and nMag under light irradiation (Figure 1c).

In fact, the present inventors have investigated the usefulness of scMagnet, using split photoactivable Cas9 endonuclease (PA-Cas9) (Non Patent Literature 4) under experimental conditions where the conventional two-molecule-type photoswitching system does not function well (e.g., in an environment with low expression levels of photoswitching proteins). As a result, it was demonstrated that the activity of scPA-Cas9, a single-molecule-system photoswitching protein, exhibited 10 times or more the photoinduction efficiency under light irradiation, compared to the activity of PA-Cas9, a two-molecule-system photoswitching protein, and that the leak activity (activity that generates even in the dark) was extremely low. Furthermore, the scMagnet system efficiently induced not only the activity of split Cas9 endonuclease, but also the activities of split Cre recombinase, split Flp recombinase, split Vav2 (guanine nucleotide exchange factor), etc. in a light-dependent manner (i.e., the scMagnet system induced light irradiation-dependent activation with a highly dynamic range of 100 times or more), with almost no leak activity.

Therefore, the scMagnet system of the present invention is a highly versatile system for optogenetically controlling the rebinding and dissociation of various split proteins.

Moreover, it has been confirmed that the proteins that constitute scMagnet (e.g., pMag and nMag) may be linked to each other by a peptide linker, that this linker portion may be a protein, and that one or several amino acid residues at the terminus on the linking side of each protein may be deleted.

As described above, the scMagnet system developed by the present inventors is a groundbreaking system that functions extremely efficiently even in an environment where the conventional Magnet systems did not function well, and can be used for a variety of purposes when it is combined with proteins other than Magnet, such as a fluorescent protein.

The present invention has been completed based on the above findings.

Specifically, the present invention includes the following (1) to (9).
(1) A protein, in which the C-terminus of the following protein (a) is directly or indirectly linked to the N-terminus of the following protein (b):
   (a) a protein comprising an amino acid sequence having a sequence identity of 80% or more to an amino acid sequence comprising a deletion of an X number of consecutive amino acid residues from the C-terminal residue to the N-terminal side of the amino acid sequence as set forth in SEQ ID No: 1 or SEQ ID No: 36, wherein X represents an integer of 0 or greater and 6 or smaller; and
   (b) a protein comprising an amino acid sequence having a sequence identity of 80% or more to an amino acid sequence comprising a deletion of a Y number of consecutive amino acid residues from the N-terminal residue to the C-terminal side of the amino acid sequence as set forth in SEQ ID No: 1 or SEQ ID No: 36, wherein Y represents an integer of 0 or greater and 55 or smaller,
   provided that when X of the protein (a) is 0, Y of the protein (b) is an integer of 1 or greater and 55 or smaller, and when Y of the protein (b) is 0, X of the protein (a) is an integer of 1 or greater and 6 or smaller.
(2) The protein according to the above (1), wherein, in the amino acid sequence of the protein (a), when the amino acids that correspond to positions 52 and 55 on the amino acid sequence as set forth in SEQ ID No: 1 are substituted with arginine, or when the amino acids that correspond to positions 16 and 19 on the amino acid sequence as set forth in SEQ ID No: 36 are substituted with arginine, in the amino acid sequence of the protein (b), the amino acid that corresponds to position 52 on the amino acid sequence as set forth in SEQ ID No: 1 is substituted with aspartic acid and the amino acid that corresponds to position 55 thereon is substituted with glycine, or the amino acid that corresponds to position 16 on the amino acid sequence as set forth in SEQ ID No: 36 is substituted with aspartic acid and the amino acid that corresponds to position 19 thereon is substituted with glycine.
(3) The protein according to the above (1), wherein the C-terminus of the protein (a) is linked to the N-terminus of the protein (b) via a polypeptide.
(4) The protein according to the above (3), wherein the polypeptide is a fluorescent protein or a bioluminescent protein.
(5) The protein according to the above (4), wherein the fluorescent protein is any one of BFP, TagBFP, mTagBFP2, mBlueberry2, mCerulean, mTurquoise, ECFP, TagCFP, Rosmarius, meleCFP, mTFP1, KCY, meffCFP, GFP, YFP, Venus, mCherry, and iRFP.
(6) The protein according to the above (4), wherein the bioluminescent protein is any one of NanoLu, Gaussia luciferase, Renilla luciferase, and firefly luciferase.
(7) A nucleic acid encoding the protein according to any one of the above (1) to (6).
(8) A method for controlling the distance between two proteins,
   in which, in the protein according to any one of the above (1) to (6), the method comprises a step of binding the protein (a) to one of the two proteins, a step of binding the protein (b) to the other of the two proteins, and irradiating the protein according to any one of the above (1) to (6) with a light.
(9) The method according to the above (8), wherein the two proteins are interacting proteins.

It is to be noted that the preposition "to" used in the present description indicates a numerical value range including the numerical values located left and right of the preposition.

### Advantageous Effects of Invention

By using the photoswitching protein of the present invention, it is possible to extremely efficiently control the binding and dissociation of proteins in an environment where conventional photoswitching proteins do not function. Furthermore, the photoswitching protein of the present invention can be used for a variety of purposes, by addition of the functions of other proteins (e.g., a fluorescent protein), as well as the binding and dissociation of proteins.

### Brief Description of Drawings

[Figure 1] Figure 1 explains the mechanism of action of scMagnet. (a) The three-dimensional structure of scMagnet in the dark is shown. The position of the N-terminus of pMag and the position of the C-terminus of nMag are, at least, 6 nm apart from each other. (b) The three-dimensional structure of scMagnet under light irradiation is shown. The position of the N-terminus of pMag and the position of the C-terminus of nMag are within 1 nm from each other. (c) The switching mode of scMagnet, which changes from an open structure to a closed structure upon blue light irradiation, is schematically shown.
[Figure 2] Figure 2 shows the results obtained by comparing the activity of scMagnet (one-molecule system) with the activity of Magnet (two-molecule system). (a) An overview of the mechanism of action of PA-Cas9 (two-molecule system) is shown. In the dark, PA-Cas9 is split into two fragments having no catalytic activity against the target genomic region. Upon blue light irradiation, pMag and nMag are dimerized in a light-dependent manner, and the binding between the N-terminal side fragment of the split Cas9 and the C-terminal fragment thereof is induced. As a result, the structure of PA-Cas9 is complemented, its activity is restored, and a double-strand cleavage is introduced into genomic DNA. (b) The frequency (%) of light-inducible indel mutations into the human VEGFA locus in HEK293T cells catalyzed by PA-Cas9 is shown. Twenty-four hours after the seeding of the cells, the cells were transfected with PA-Cas9 and gRNA and were then maintained in the dark or under blue light irradiation (470 ± 20 nm, 1.0 W/m⁻²) for 24 hours. Thereafter, genomic DNA was recovered from the cells. The indel mutation percentage of the recovered DNA samples was measured using the TIDE assay. Data are shown as a mean value ± s.d. (n = 3, from three independent experiments). Dots represent independent data points. Full-length Cas9 and pcDNA3.1-V5-HisA (empty vector) are positive or negative controls, respectively. In each graph for the full-length Cas9, the empty vector and the PA-Cas9, the left bar represents the value in the dark, and the right bar represents the value under light irradiation. (c) An overview of the mechanism of action of scPA-Cas9 (one-molecule system) is shown. In the dark, scPA-Cas9 exists as a single polypeptide, and since the N-terminal side fragment of split Cas9 and the C-terminal side fragment thereof are located far apart from each other, scPA-Cas9 does not have catalytic activity against the target genomic region. Upon blue light irradiation, a significant light-dependent structure change occurs in the single strand-based Magnet system (scMagnet), and the binding between the N-terminal side fragment of split Cas9 and the C-terminal side fragment thereof is induced. As a result, the structure of PA-Cas9 is complemented, its activity is restored, and a double-strand cleavage is introduced into genomic DNA. (d) The frequency (%) of light-inducible indel mutations into the human VEGFA locus in HeLa cells catalyzed by scPA-Cas9 or PA-Cas9 is shown. Twenty-four hours after the seeding of the cells, the cells were transfected with scPA-Cas9 or PA-Cas9 and gRNA and were then maintained in the dark or under blue light irradiation (470 ± 20 nm, 1.0 W/m⁻²) for 48 hours. Thereafter, genomic DNA was recovered from the cells. The indel mutation percentage of the recovered DNA samples was measured using the TIDE assay. Data are shown as a mean value ± s.d. (n = 3, from three independent experiments). Dots represent independent data points. Full-length Cas9 and pcDNA3.1-V5-HisA (empty vector) are positive or negative controls, respectively. In each graph for the full-length Cas9, the empty vector, PA-Cas9 and scPA-Cas9, the left bar represents the value in the dark, and the right bar represents the value under light irradiation.

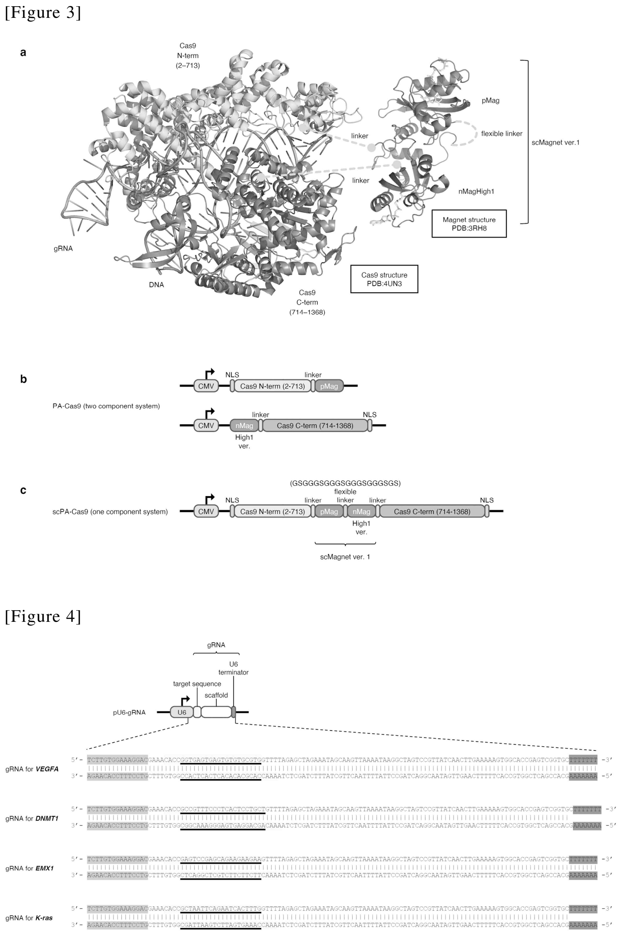

[Figure 3] Figure 3 explains the structure of scPA-Cas9 and the construct used for preparing it. (a) A schematic view of scPA-Cas9, based on the crystal structures of Cas9, gRNA, and target DNA (PDB: 4UN3) and the crystal structure of a dimer of Vivid under light irradiation, which is the precursor protein of the Magnet system (pMag and nMag) (PDB: 3RH8), is shown. (b) A schematic view of the expression construct of PA-Cas9 is shown. CMV indicates a cytomegalovirus promoter and NLS indicates a nuclear localization signal, respectively. (c) A schematic view of the expression construct of scPA-Cas9 is shown.
[Figure 4] Figure 4 shows the expression constructs of a gRNA expression vector and the nucleotide sequences thereof. gRNAs targeting human VEGFA, human DNMT1, human EMX1, and mouse K-ras were prepared by an annealed oligo cloning method using the BstI site of a pU6-gRNA expression vector (Addgene: 47108).

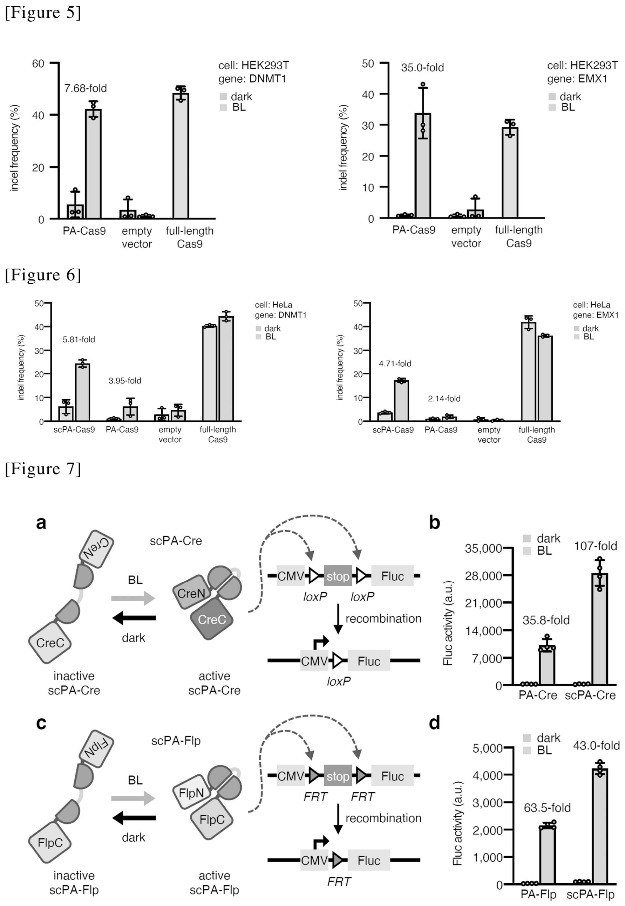

[Figure 5] Figure 5 shows the percentage of indel mutations introduced into the human DNMT1 and human EMX1 loci in HEK293T cells by PA-Cas9. Twenty-four hours after the seeding of the cells, the cells were transfected with PA-Cas9 and gRNA and were then maintained in the dark or under blue light irradiation (470 ± 20 nm, 1.0 W/m⁻²) for 24 hours. The indel mutation percentage of the genomic DNA recovered from the cells was measured using the TIDE assay. Data are shown as a mean value ± s.d. (n = 3, from three independent experiments). Dots represent independent data points. Full-length Cas9 and pcDNA3.1-V5-HisA (empty vector) are positive or negative controls, respectively. In each graph for the PA-Cas9, the empty vector and the full-length Cas9, the left bar represents the value in the dark, and the right bar represents the value under light irradiation.
[Figure 6] Figure 6 shows the percentage of indel mutations introduced into the human DNMT1 and human EMX1 loci in HeLa cells by scPA-Cas9 and PA-Cas9. Twenty-four hours after the seeding of the cells, the cells were transfected with scPA-Cas9 or PA-Cas9 and gRNA and were then maintained in the dark or under blue light irradiation (470 ± 20 nm, 1.0 W/m⁻²) for 48 hours. The indel mutation percentage of the genomic DNA recovered from the cells was measured using the TIDE assay. Data are shown as a mean value ± s.d. (n = 3, from three independent experiments). Dots represent independent data points. Full-length Cas9 and pcDNA3.1-V5-HisA (empty vector) are positive or negative controls, respectively. In each graph for the scPA-Cas9, the PA-Cas9, the empty vector and the full-length Cas9, the left bar represents the value in the dark, and the right bar represents the value under light irradiation.
[Figure 7] Figure 7 shows the versatility of the scMagnet system. (a) An outline of the mechanism of action of scPA-Cre is shown. scPA-Cre activated by blue light irradiation causes recombination of a DNA sequence flanked by two loxP sites. CMV indicates a cytomegalovirus promoter, stop indicates a polyadenylation transcription "stop" signal sequence, and Fluc indicates firefly luciferase. (b) Comparison of scPA-Cre (one-molecule system) and PA-Cre (two-molecule system) by luciferase assay is shown. COS-7 cells transiently transfected with scPA-Cre or PA-Cre and a loxP-stop-loxP Fluc reporter were subjected to luciferase assay using Fluc expression as an indicator either in the dark or under blue light irradiation (470 ± 20 nm, 1.0 W/m⁻²) for 24 hours. The percentage of scPA-Cre-encoding DNA or PA-Cre-encoding DNA to the total transfection amount was set to 4.8%. Data are shown as a mean value ± s.d. (n = 4, from three independent experiments). Dots represent independent data points. In each graph for the PA-Cre and the scPA-Cre, the left bar represents the value in the dark, and the right bar represents the value under light irradiation. (c) An outline of the mechanism of action of scPA-Flp is shown. scPA-Flp activated by blue light irradiation causes recombination of a DNA sequence flanked by two FRT sites. (d) Comparison of scPA-Flp (one-molecule system) and PA-Flp (two-molecule system) by luciferase assay is shown. COS-7 cells transiently transfected with scPA-Flp or PA-Flp and an FRT-stop-FRT Fluc reporter were subjected to luciferase assay using Fluc expression as an indicator either in the dark or under blue light irradiation (470 ± 20 nm, 1.0 W/m⁻²) for 24 hours. The percentage of scPA-Cre-encoding DNA or PA-Cre-encoding DNA to the total transfection amount was set to 4.8%. Data are shown as a mean value ± s.d. (n = 4, from three independent experiments). Dots represent independent data points. In each graph for the PA-Flp and the scPA-Flp, the left bar represents the value in the dark, and the right bar represents the value under light irradiation.

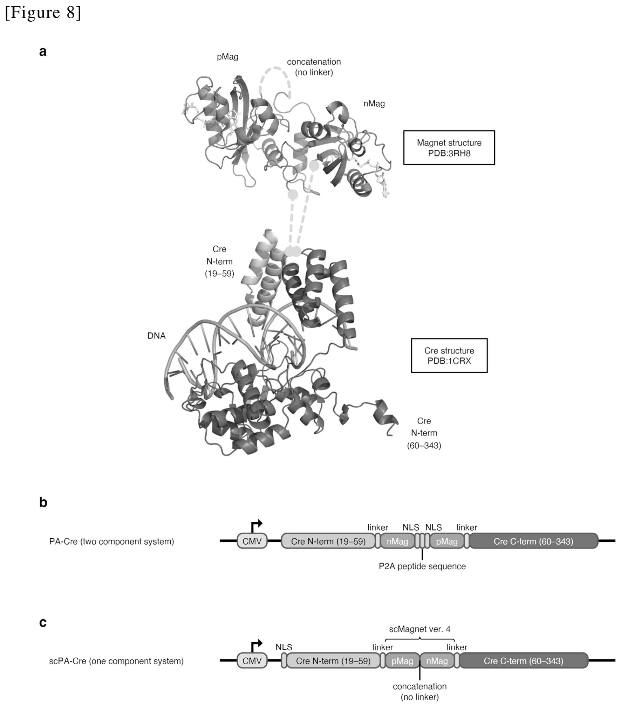

[Figure 8] Figure 8 explains the structure of scPA-Cre and the construct used for preparing it. (a) A schematic view of scPA-Cre, based on the crystal structure of Cre recombinase (PDB: 1CRX) and the crystal structure of a dimer of Vivid (PDB: 3RH8) under light irradiation, is shown. (b) A schematic view of the expression construct of PA-Cre is shown. CMV indicates a cytomegalovirus promoter, NLS indicates a nuclear localization signal, and P2A indicates a self-cleaving peptide, respectively. (c) A schematic view of the expression construct of scPA-Cre is shown.

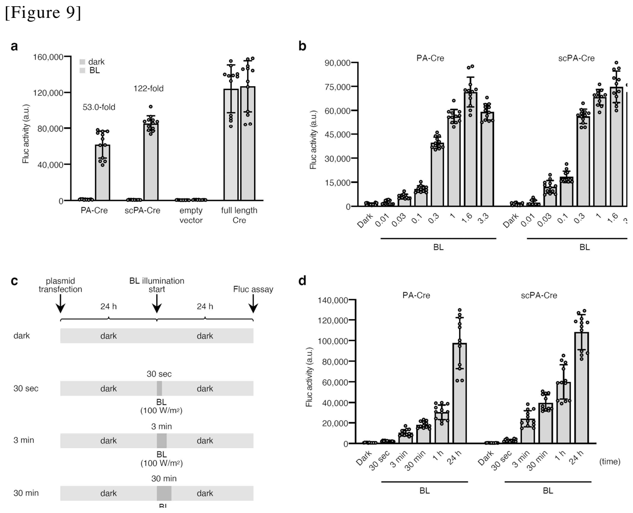

[Figure 9] Figure 9 shows the results obtained by evaluating the activity of scPA-Cre. (a) Comparison of scPA-Cre and PA-Cre by luciferase assay is shown. COS-7 cells transiently transfected with scPA-Cre or PA-Cre and a loxP-stop-loxP Fluc reporter were subjected to luciferase assay using Fluc expression as an indicator either in the dark or under blue light irradiation (470 ± 20 nm, 1.0 W/m⁻²) for 24 hours. The percentage of scPA-Cre-encoding DNA or PA-Cre-encoding DNA to the total transfection amount was set to 9.1%. Data are shown as a mean value ± s.d. (n = 12, from three independent experiments). Dots represent independent data points. Full-length Cre and pcDNA3.1 (empty vector) are positive or negative controls, respectively. In each graph for the PA-Cre, the scPA-Cre, the empty vector and the full-length Cre, the left bar represents the value in the dark, and the right bar represents the value under light irradiation. (b) Light intensity dependence of scPA-Cre and PA-Cre is shown. COS-7 cells transiently transfected with scPA-Flp or PA-Flp and a loxP-stop-loxP Fluc reporter were subjected to luciferase assay using Fluc expression as an indicator either in the dark or under blue light irradiation (470 ± 20 nm, 0.01 to 3.3 W/m⁻²) for 24 hours. The percentage of scPA-Cre-encoding DNA or PA-Cre-encoding DNA to the total transfection amount was set to 9.1%. Data are shown as a mean value ± s.d. (n = 12, from three independent experiments). Dots represent independent data points. (c) A view showing experimental schedule regarding short-term irradiation and continuous irradiation with a blue light. (d) Comparison of the response of scPA-Cre or PA-Cre under different blue light irradiation times (100 W/m⁻², 30 sec to 3 min; 1.0 W/m⁻², 30 min to 24 h). Data are shown as a mean value ± s.d. (n = 12, from three independent experiments). Dots represent independent data points.

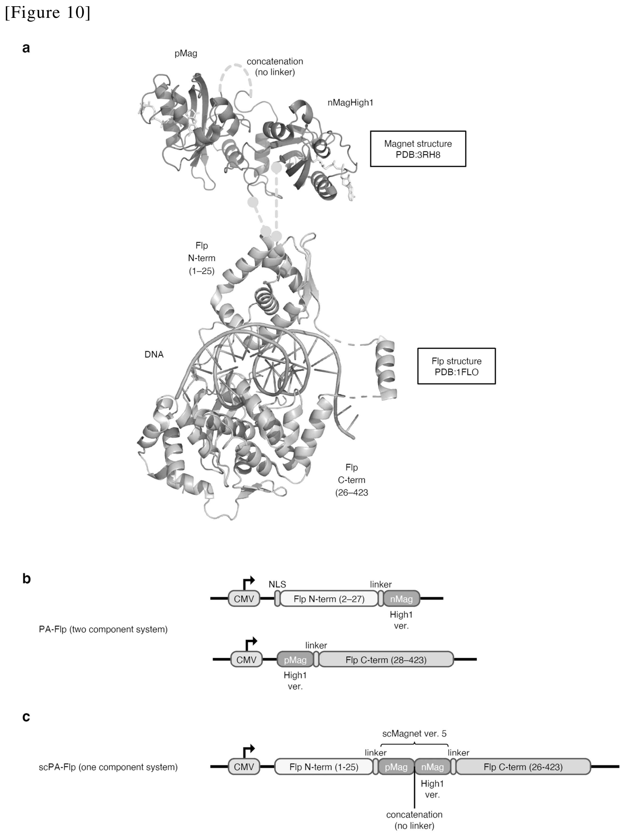

[Figure 10] Figure 10 explains the structure of scPA-Flp and the construct used for preparing it. (a) A schematic view of scPA-Flp, based on the crystal structure of Flp recombinase (PDB: 1FLO) and the crystal structure of a dimer of Vivid (PDB: 3RH8) under light irradiation, is shown. (b) A schematic view of the expression construct of PA-Flp is shown. pMagHigh1 is a magnet in the Magnet system, and has high affinity and long light cycle. CMV indicates a cytomegalovirus promoter, and NLS indicates a nuclear localization signal, respectively. (c) A schematic view of the expression construct of scPA-Flp is shown.

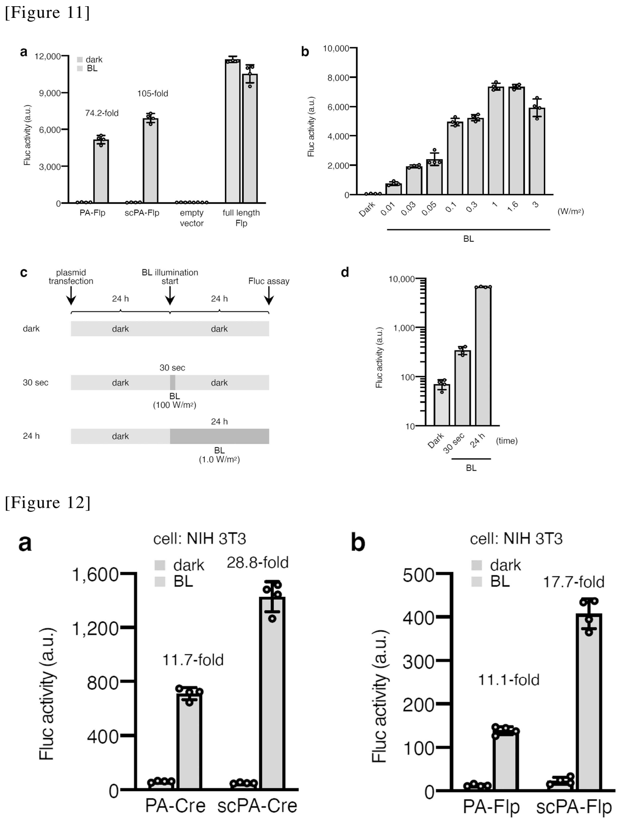

[Figure 11] Figure 11 shows the results obtained by evaluating the activity of scPA-Flp. (a) Comparison of scPA-Flp and PA-Flp by luciferase assay is shown. COS-7 cells transiently transfected with scPA-Flp or PA-Flp and an FRT-stop-FRT Fluc reporter were subjected to luciferase assay using Fluc expression as an indicator either in the dark or under blue light irradiation (470 ± 20 nm, 1.0 W/m⁻²) for 24 hours. The percentage of scPA-Flp-encoding DNA or PA-Flp-encoding DNA to the total transfection amount was set to 9.1%. Data are shown as a mean value ± s.d. (n = 4, from three independent experiments). Dots represent independent data points. Full-length Flp and pcDNA3.1 (empty vector) are positive or negative controls, respectively. In each graph for the PA-Flp, the scPA-Flp, the empty vector and the full-length Flp, the left bar represents the value in the dark, and the right bar represents the value under light irradiation. (b) Light intensity dependence of scPA-Flp and PA-Flp is shown. COS-7 cells transiently transfected with scPA-Flp or PA-Flp and an FRT-stop-FRT Fluc reporter were subjected to luciferase assay using Fluc expression as an indicator either in the dark or under blue light irradiation (470 ± 20 nm, 0.01 to 3.3 W/m⁻²) for 24 hours. Data are shown as a mean value ± s.d. (n = 4, from three independent experiments). Dots represent independent data points. (c) A view showing experimental schedule regarding short-term irradiation and continuous irradiation with a blue light. (d) Comparison of the response of scPA-Flp or PA-Flp under different blue light irradiation times (100 W/m⁻², 30 sec to 3 min; 1.0 W/m⁻², 30 min to 24 h). Data are shown as a mean value ± s.d. (n = 4, from three independent experiments). Dots represent independent data points. In each graph for the PA-Flp, the scPA-Flp and the full-length Flp, the left bar represents the value in the dark, and the right bar represents the value under light irradiation.
[Figure 12] Figure 12 shows the results obtained by evaluating the activities of scPA-Cre and scPA-Flp in NIH3T3 cells. (a) Comparison of scPA-Cre and PA-Cre by luciferase assay. NIH3T3 cells transiently transfected with scPA-Cre or PA-Cre and a loxP-stop-loxP Fluc reporter were subjected to luciferase assay using Fluc expression as an indicator either in the dark or under blue light irradiation (470 ± 20 nm, 1.0 W/m⁻²) for 24 hours. The percentage of scPA-Cre-encoding DNA or PA-Cre-encoding DNA to the total transfection amount was set to 4.8%. Data are shown as a mean value ± s.d. (n = 4, from three independent experiments). Dots represent independent data points. In each graph for the PA-Cre and the scPA-Cre, the left bar represents the value in the dark, and the right bar represents the value under light irradiation. (b) Comparison of scPA-Flp and PA-Flp by luciferase assay. NIH3T3 cells transiently transfected with scPA-Flp or PA-Flp and an FRT-stop-FRT Fluc reporter were subjected to luciferase assay using Fluc expression as an indicator either in the dark or under blue light irradiation (470 ± 20 nm, 1.0 W/m⁻²) for 24 hours. The percentage of scPA-Cre-encoding DNA or PA-Cre-encoding DNA to the total transfection amount was set to 4.8%. Data are shown as a mean value ± s.d. (n = 4, from three independent experiments). Dots represent independent data points. In each graph for the PA-Flp and the scPA-Flp, the left bar represents the value in the dark, and the right bar represents the value under light irradiation.

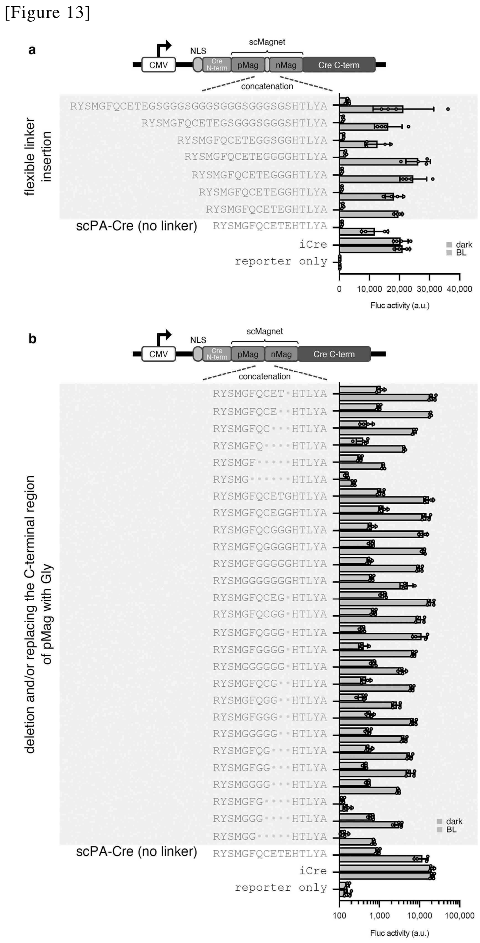

[Figure 13] Figure 13 shows the results obtained by studying the influence of a linker and the amino acid deletion of pMAg in scMagnet upon the activity thereof. (a) The results obtained by comparing the activity of directly linked scPA-Cre with the activity of scPA-Cre linked with linkers of various lengths by luciferase assay are shown. (b) The results obtained by comparing the activity of directly linked scPA-Cre with the activity of scPA-Cre mutants in which 1 to 6 amino acids were deleted on the C-terminal side of pMag and linked with linkers of various lengths by luciferase assay are shown. COS-7 cells transiently transfected with scPA-Cre or the scPA-Cre mutants and a loxP-stop-loxP Fluc reporter were subjected to luciferase assays using Fluc expression as an indicator in the dark or under blue light irradiation (470 ± 20 nm, 1.0 W/m⁻²) for 24 hours. Data are shown as a mean value ± s.d. (n = 4, from three independent experiments). Dots represent independent data points. In each graph for the scPA-Cre or the scPA-Cre mutants, the upper bar indicates the value in the dark, and the lower bar indicates the value under light irradiation.

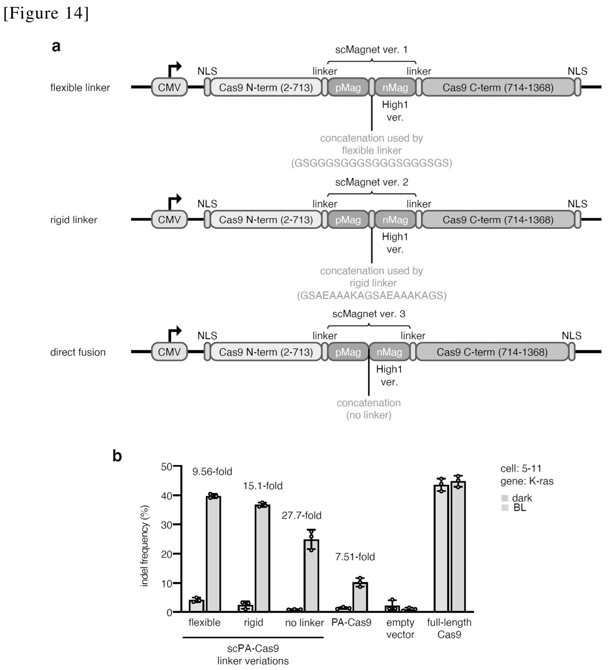

[Figure 14] Figure 14 shows the results obtained by studying the suitability of the scMagnet linker using scPA-Cas9. (a) A flexible linker (GSGGGSGGGSGGGSGGGSGS; SEQ ID No: 34) or a rigid linker (GSAEAAAKAGSAEAAAKAGS; SEQ ID No: 35) was inserted between pMag and nMagHigh1. "No linker" indicates a direct link between pMag and nMagHigh1. CMV (cytomegalovirus promoter) indicates the cytomegalovirus promoter, and NLS (nuclear localization signal) indicates the nuclear localization signal. (b) Measurement of light-inducible indel mutations by scPA-Cas9 and PA-Cas9 at the mouse K-ras locus in 5-11 cells (a cell line derived from pancreatic cancer in LSL-^{KrasG12D/+}; LSL- Trp53^{Rl72H/+}; ptfla-Cre⁻²) for 48 hours. The indel mutation percentage of genomic DNA recovered from the cells was measured by TIDE assay. Data are shown as a mean value ± s.d. (n = 3, from three independent experiments). Dots represent independent data points. full-length Cas9 and pcDNA3.1-V5-HisA are positive or negative controls, respectively. In each graph for the scPA-Cas9 (flexible, rigid, and no linker), the PA-Cas9, the empty vector, and the full-length Cas9, the left bar represents the value in the dark, and the right bar represents the value under light irradiation.

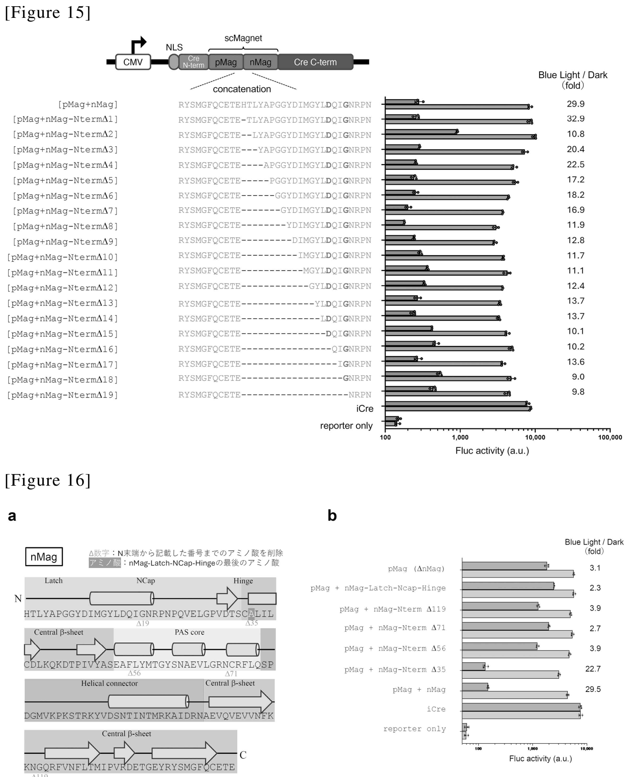

[Figure 15] Figure 15 shows the results obtained by studying the influence of the amino acid deletion of nMag in scMagnet on the activity thereof. The results obtained by comparing the activity of directly linked scPA-Cre with the activity of scPA-Cre mutants in which 1 to 19 amino acids on the N-terminal side of nMag were deleted and linked with pMag by luciferase assay are shown. COS-7 cells transiently transfected with scPA-Cre or the scPA-Cre mutants and a loxP-stop-loxP Fluc reporter were subjected to luciferase assays using Fluc expression as an indicator in the dark or under blue light irradiation (470 ± 20 nm, 1.0 W/m⁻²) for 24 hours. Data are shown as a mean value ± s.d. (n = 4, from three independent experiments). Dots represent independent data points. In each graph for the scPA-Cre or the scPA-Cre mutants, the upper bar indicates the value in the dark, and the lower bar indicates the value under light irradiation.
[Figure 16] Figure 16 shows the results obtained by examining the Cre recombination activity of scPA-Cre mutants introduced with nMag, which contains stepwise deletions of amino acids on the N-terminal side. There were prepared a scPA-Cre mutant introduced with an nMag mutant having only the Latch-NCap-Hinge, and scPA-Cre mutants introduced with nMag mutants having deletions of amino acids on the N-terminal side up to the amino acid residue 119 (Δ119), up to the amino acid residue 71 (Δ71), up to the amino acid residue 56 (Δ56), or up to the amino acid residue 35 (Δ35). Then, Cre recombination activity was measured for these mutants. (a) shows a schematic view of the structure of each nMag mutant, and (b) shows the results obtained by measuring Cre recombination activity. COS-7 cells were transiently transfected with scPA-Cre (pMag+nMag) or the scPA-Cre mutants and a loxP-stop-loxP Fluc reporter, and were then subjected to luciferase assays using Fluc expression as an indicator in the dark or under blue light irradiation (470 ± 20 nm, 1.0 W/m⁻²) for 24 hours. Data are shown as a mean value ± s.d. (n = 4, from three independent experiments). Dots represent independent data points. In the graph of (b), the upper bar indicates the value in the dark, and the lower bar indicates the value under light irradiation.

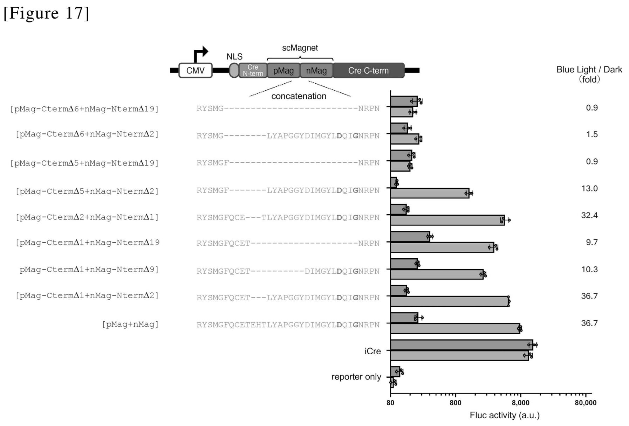

[Figure 17] Figure 17 shows the results obtained by examining the Cre recombination activity of scPA-Cre mutants introduced with various combinations of mutants, in which the C-terminus of pMag and the N-terminus of nMag were each partially deleted, and the pMag and the nMag were then linked to each other. pMag mutants deleting one amino acid residue (pMag-CtermΔ1), two amino acid residues (pMag-CtermΔ2), five amino acid residues (pMag-CtermΔ5), or six amino acid residues (pMag-CtermΔ6) on the C-terminal side of pMag, were linked to nMag mutants deleting one amino acid residue (nMag-NtermΔ1), two amino acid residues (nMag-NtermΔ2), nine amino acid residues (nMag-NtermΔ9), or 19 amino acid residues (nMag-NtermΔ19) on the N-terminal side, thereby preparing various types of scPA-Cre mutants. Then, Cre recombination activity was measured for these mutants. COS-7 cells were transiently transfected with scPA-Cre (pMag+nMag) or the scPA-Cre mutants and a loxP-stop-loxP Fluc reporter, and were then subjected to luciferase assays using Fluc expression as an indicator in the dark or under blue light irradiation (470 ± 20 nm, 1.0 W/m⁻²) for 24 hours. Data are shown as a mean value ± s.d. (n = 4, from three independent experiments). Dots represent independent data points. In the graph, the upper bar indicates the value in the dark, and the lower bar indicates the value under light irradiation.

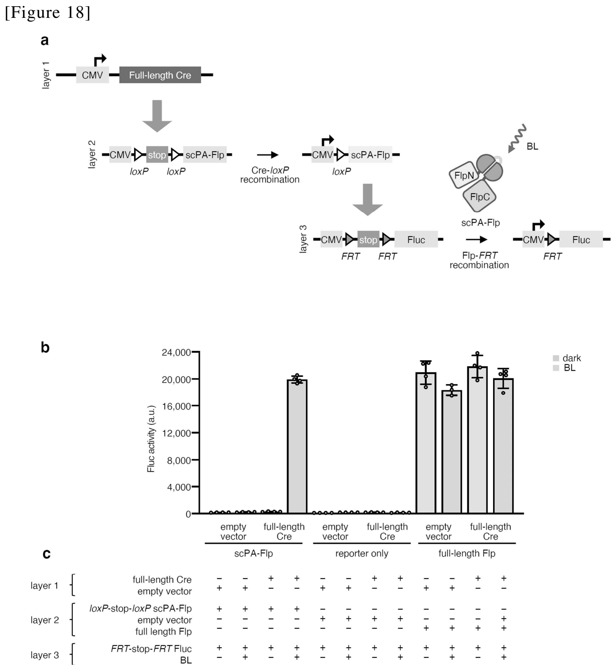

[Figure 18] Figure 18 shows the results obtained by evaluating a light-dependent recombination cascade, in which full-length Cre is combined with scPA-Flp. (a) An outline of the recombination cascade using multiple gene expression steps is shown. (b) COS-7 cells transfected with the cDNAs shown in (c) were maintained in the dark or under blue light irradiation (470 ± 20 nm, 1.0 W/m⁻²) for 24 hours, and were then subjected to a luciferase assay. Data are shown as a mean value ± s.d. (n = 4, from three independent experiments). Dots represent independent data points. Full-length Cas9 and pcDNA3.1-V5-HisA are positive or negative controls, respectively. In each graph for the empty vector and the full-length Cre, the left bar represents the value in the dark, and the right bar represents the value under light irradiation. (c) Transfection conditions for the luciferase assay shown in (b) are shown.

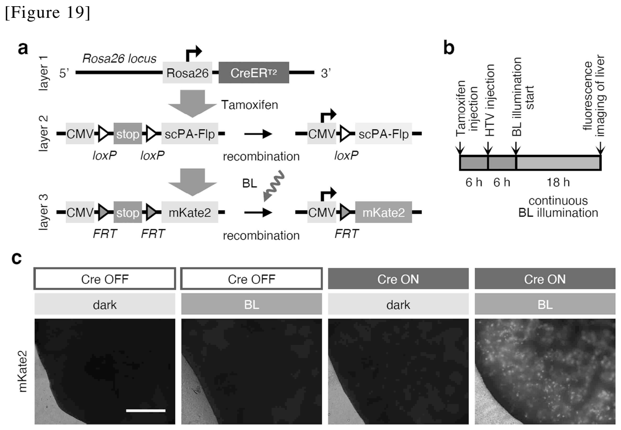

[Figure 19] Figure 19 shows the results obtained by evaluating a light-dependent recombination cascade, in which full-length Cre is combined with scPA-Flp. (a) An outline of the recombination cascade using multiple gene expression steps is shown. (b) The schedule of an in vivo experiment is shown. (c) Intracellular operational status of the recombination cascade of scPA-Flp initiated by CreER^{T2} is shown. Rosa26-CreER^{T2} knock-in mice were injected with tamoxifen or corn oil, and were then transiently injected with cDNA encoding loxP-stop-loxP scPA-Flp and an FRT-stop-FRT mKate2 reporter by an HTV (hydrodynamic tail vein) injection method. The mice were then reared in the dark or under blue light irradiation (470 ± 20 nm, 132 W/m⁻²) for 18 hours. Eighteen hours after the rearing, livers were excised from the mice, and were then subjected to fluorescence imaging. Images show typical results from three times of measurements. The scale bar is 1 mm.

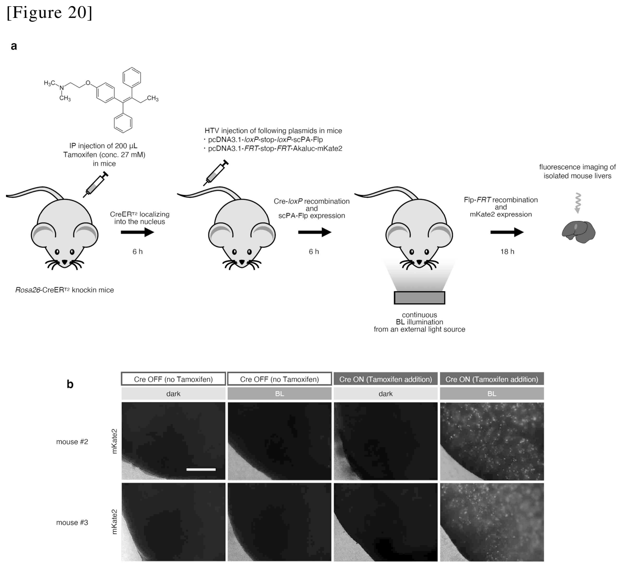

[Figure 20] Figure 20 shows the results obtained by evaluating in vivo scPA-Flp activity. (a) The experimental schedule is shown. Rosa26-CreER^{T2} knock-in mice were intraperitoneally injected with 200 µL of tamoxifen (27 mM) or corn oil, and 6 hours after the injection, the mice were injected with cDNA encoding loxP-stop-loxP scPA-Flp and an FRT-stop-FRT mKate2 reporter by an HTV (hydrodynamic tail vein) injection method. Then, from 6 hours after the injection of the cDNA, the mice were reared in the dark or under blue light irradiation (470 ± 20 nm, 132 W/m⁻²) for 18 hours. Eighteen hours after the rearing, livers were excised from the mice, and were then subjected to fluorescence imaging. (b) Images other than the images shown in Figure 16(c) are shown. The scale bar is 1 mm.

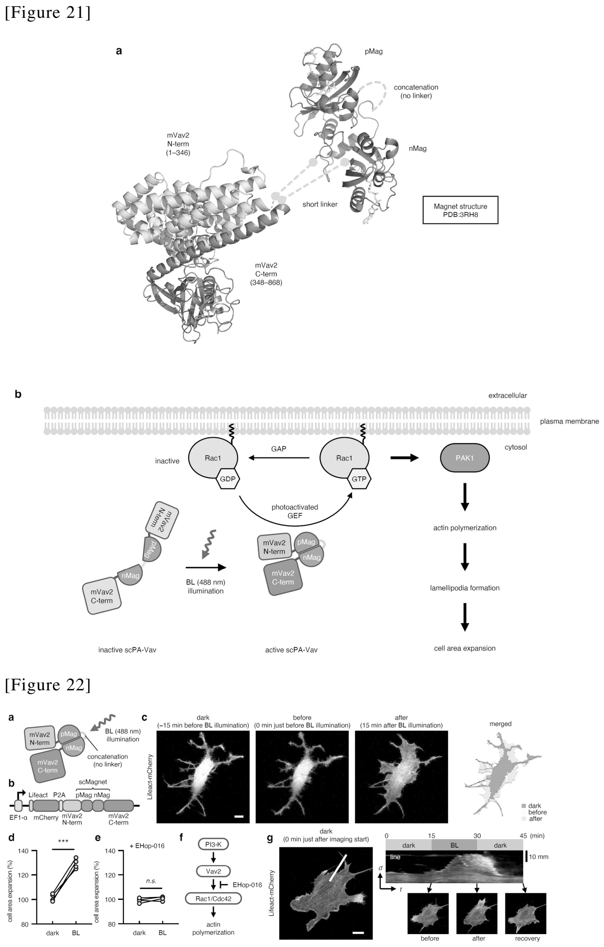

[Figure 21] Figure 21 shows schematics view of the structure and mechanism of action of scPA-Vav. (a) A schematic view of scPA-Vav based on the crystal structure of the dimer (PDB: 3RH8) of mouse Vav2 GEF9 and Vivid under light irradiation is shown. (b) It is a view schematically showing the mechanism of action of scPA-Vav in cell expansion. Vav2 is a Rho family guanine exchange factor that converts GDP to GTP and activates Rac1 GTPase in various cells. GAP indicates GTPase-activating protein, and PAK indicates p21-related kinase.
[Figure 22] Figure 22 shows the results obtained by studying the reversibility of the function of scMagnet. (a) A schematic view of scPA-Vav under blue light (BL) irradiation is shown. (b) A schematic view of an scPA-Vav construct is shown. In the expansion assay of the cell area, Lifeact-mCherry was used as a biosensor for detecting actin polymerization. P2A indicates a self-cleaving peptide. (c) The assay results of the light-dependent cell area expansion activity induced by scPA-Vav are shown. The fluorescence images of Lifeact-mCherry, and NIH 3T3 cells expressing scPA-Vav2, were taken 15 minutes before, during, and 15 minutes after the blue light irradiation. The irradiation with a blue light was performed with a 488 nm, 1 mW laser light. A binary image of the cells is shown in the right view. Typical results obtained from four independent experiments are shown. (d) The results obtained by quantifying the cell expansion region of cells expressing scPA-Vav2 are shown. Data are plotted from independent data values (n = 4, from four independent experiments). (e) The results obtained by quantifying the cell expansion region of scPA-Vav2-expressing cells in the presence of EHop-016 (Rac GTPase inhibitor, 1 µM). Data are plotted from independent data values (n = 4, from four independent experiments). (f) A view showing that EHop-016 inhibits the binding of Rac1 to Rho GEF Vav2. (g) A Kymograph of the cell area along the line indicated in the left view is shown. The results are shown as typical experimental results selected from four independent experiments. ***P < 0.001 is by Welch's two-tailed t-test. n.s. indicates no significant difference. The scale bar is 10 µm.

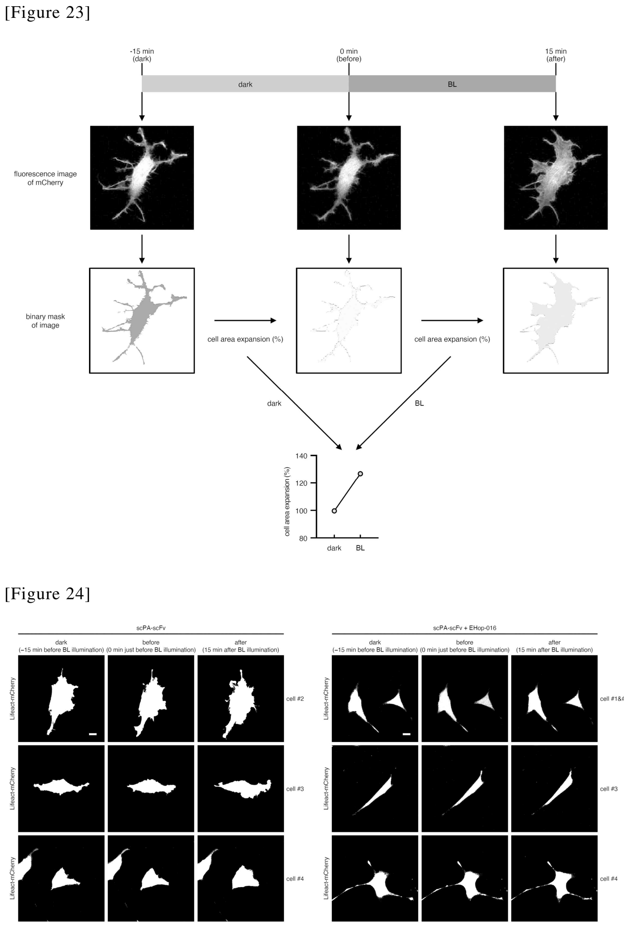

[Figure 23] Figure 23 shows the results obtained by quantifying the amount of cell expansion induced by scPA-Vav and 2PscPA-Vav. The fluorescence images of Lifeact-mCherry, and NIH 3T3 cells expressing scPA-Vav or 2PscPA-Vav, were taken 15 minutes before (dark), during (before), and 15 minutes after (after) the blue light irradiation. The blue light irradiation was performed with a 488 nm, 1 mW laser light for a one-photon excitation experiment and with an 808 nm, 25 mW laser light for a two-photon excitation experiment. A binary image of the cells is shown in the right view. Typical results obtained from four independent experiments are shown. The fluorescence images were converted to binary data using ImageJ software. Using the binary data, the percentage of the "dark" cell area to the "before" cell area and the percentage of the "before" cell area to the "after" cell area were calculated.
[Figure 24] Figure 24 shows the results obtained by analyzing the expansion of a light-dependent cell area induced by scPA-Vav. The fluorescence images of Lifeact-mCherry, and NIH 3T3 cells expressing scPA-Vav, were taken 15 minutes before, during, and 15 minutes after the blue light irradiation. The blue light irradiation was performed with a 488 nm, 1 mW laser light. The fluorescence images were converted to binary data using ImageJ software. Using the binary data, the percentage (dark) of the cell area before the irradiation to the cell area in the dark and the percentage (BL) of the cell area after the irradiation to the cell area before the irradiation were calculated. The calculated cell expansion percentages were used to produce the graphs shown in Figure 22d and e. All scale bars are 10 µm.

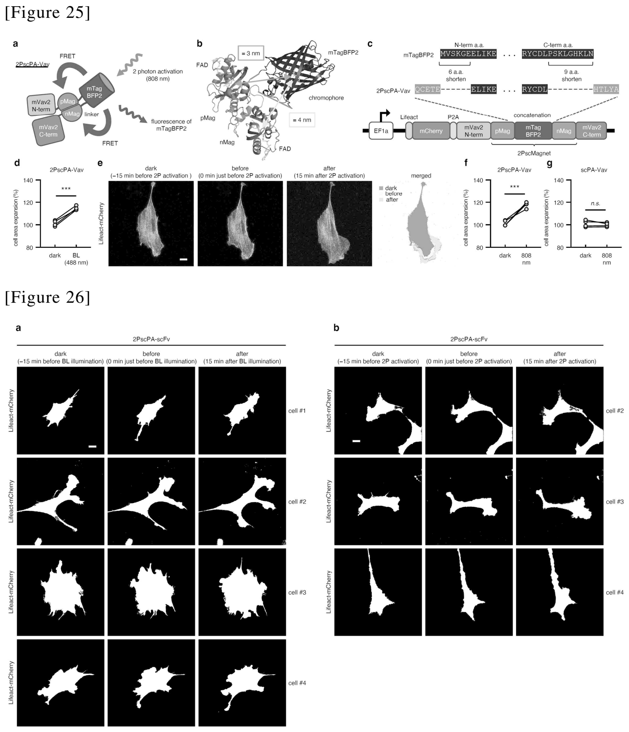

[Figure 25] Figure 25 shows the results obtained by studying the operability of the scMagnet system by two-photon excitation. (a) A schematic view of the mechanism of action of 2PscMagnet is shown. mTagBFP2 was inserted between pMag and nMag (2PscMagnet). 2PscMagnet can activate 2PscMagnet by two-photon excitation via fluorescence resonance energy transfer (FRET) from mTagBFP2 to flavin adenine dinucleotide (FAD) binding to pMag and nMag. (b) The three-dimensional structure of 2PscMagnet is shown. The distances between the chromophore of mTagBFP2 (FERT donor) and the FAD (FERT acceptor) of pMag, and between the chromophore of the FERT donor and the FAD (FERT acceptor) of nMag, are approximately 3 nm and 4 nm, respectively. These distances are close enough for FRET to occur. (c) A schematic view of the structure of 2PscPA-Vav. Based on the crystal structure information, the N-terminal amino acid residues (a.a. 1-6) and C-terminal amino acid residues (a.a. 229-237) of mTagBPF2 were maximally shortened and inserted into scMagnet. (d) The results obtained by quantifying the expanded region of 2PscPA-Vav-expressing cells expanded by one-photon excitation are shown. Light-dependent cell area expansion was induced by irradiation with a 488 nm, 1 mW laser light. Data are plotted from independent data values (n = 4, from four independent experiments). (e) Light-dependent cell area expansion was induced by two-photon (2P) excitation of 2PsPA-Vav. The fluorescence images of Lifeact-mCherry, and NIH 3T3 cells expressing 2PscPA-Vav2, were taken 15 minutes before, during, and 15 minutes after two-photon excitation. The two-photon excitation was performed using an 808-nm, 25-mW laser light. A binary image of the cells is shown in the right view. Typical results obtained from four independent experiments are shown. (f and g) The results obtained by quantifying the expanded region of cells expressing 2PscPA-Vav2 (f) or scPA-Vav2 (g) are shown. The light-dependent cell area expansion was induced by 2PsPA-Vav or scPA-Vav2 using an 808-nm, 25-mW laser light. Data are plotted from independent data values (n = 4, from four independent experiments). ***P < 0.001 is by Welch's two-tailed t-test. n.s. indicates no significant difference. The scale bar is 10 µm.
[Figure 26] Figure 26 shows the results obtained by analyzing light-dependent cell area expansion induced by 2PscPA-Vav. The fluorescence images of Lifeact-mCherry, and NIH 3T3 cells expressing 2PscPA-Vav, were taken 15 minutes before (dark), during (before), and 15 minutes after (after) the light irradiation. The light irradiation was performed with a 488 nm, 1 mW laser light for one-photon excitation (a) and with an 808 nm, 25 mW laser light for two-photon excitation (b). The fluorescence images were converted to binary data using ImageJ software. Using the binary data, the percentage of the "dark" cell area to the "before" cell area and the percentage of the "before" cell area to the "after" cell area were calculated. The calculated cell expansion percentages were used to produce the graphs shown in Figure 25d and f. All scale bars are 10 µm.

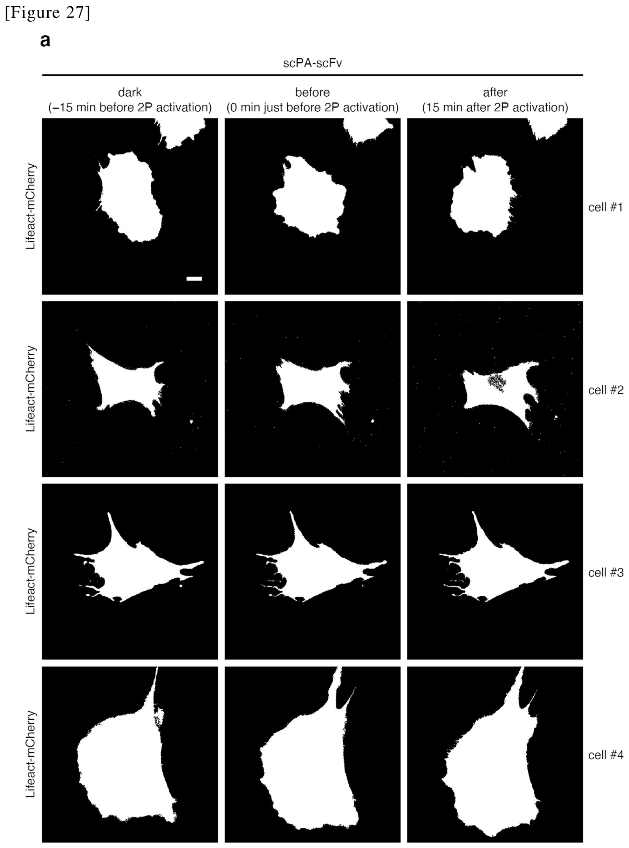

[Figure 27] Figure 27 shows the results obtained by analyzing light-dependent cell area expansion induced by scPA-Vav. The fluorescence images of Lifeact-mCherry, and NIH 3T3 cells expressing scPA-Vav, were taken 15 minutes before (dark), during (before), and 15 minutes after (after) the light irradiation. The light irradiation was performed with an 808 nm, 25 mW laser light. The fluorescence images were converted to binary data using ImageJ software. Using the binary data, the percentage of the "dark" cell area to the "before" cell area and the percentage of the "before" cell area to the "after" cell area were calculated. The calculated cell expansion percentages were used to produce the graph shown in Figure 25g. All scale bars are 10 µm.

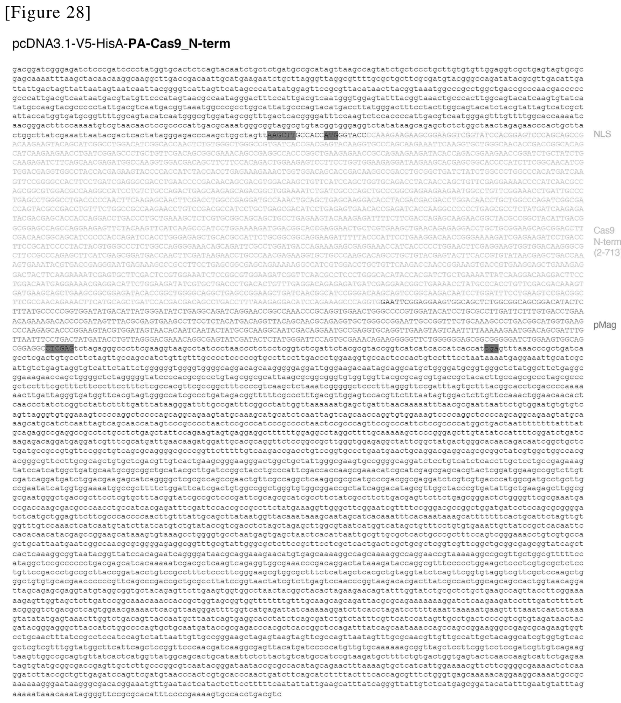

[Figure 28] Figure 28 shows the nucleotide sequence (SEQ ID No: 18) of pcDNA3.1-V5-HisA-PA-Cas9_N-term.

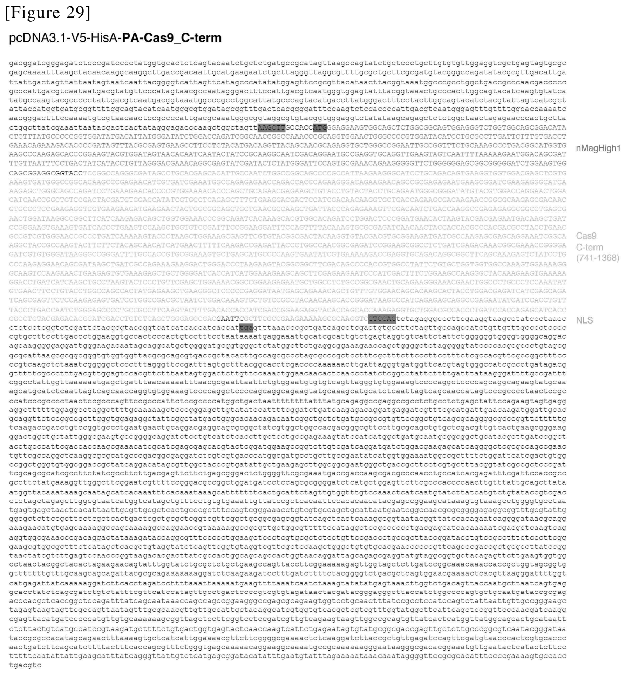

[Figure 29] Figure 29 shows the nucleotide sequence (SEQ ID No: 19) of pcDNA3.1-V5-HisA-PA-Cas9_C-term.

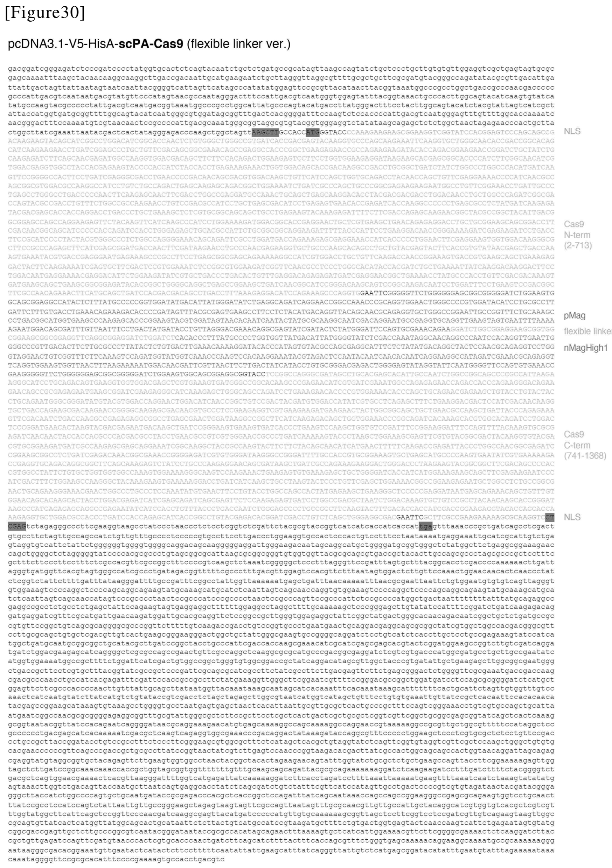

[Figure 30] Figure 30 shows the nucleotide sequence (SEQ ID No: 20) of pcDNA3.1-V5-HisA-scPA-Cas9 (flexible linker).

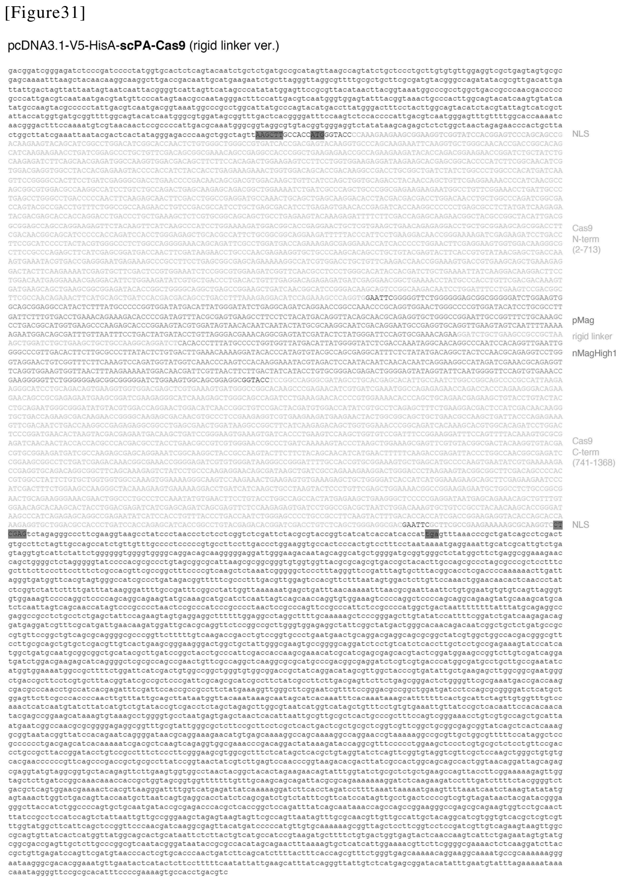

[Figure 31] Figure 31 shows the nucleotide sequence (SEQ ID No: 21) of pcDNA3.1-V5-HisA-scPA-Cas9 (rigid linker).

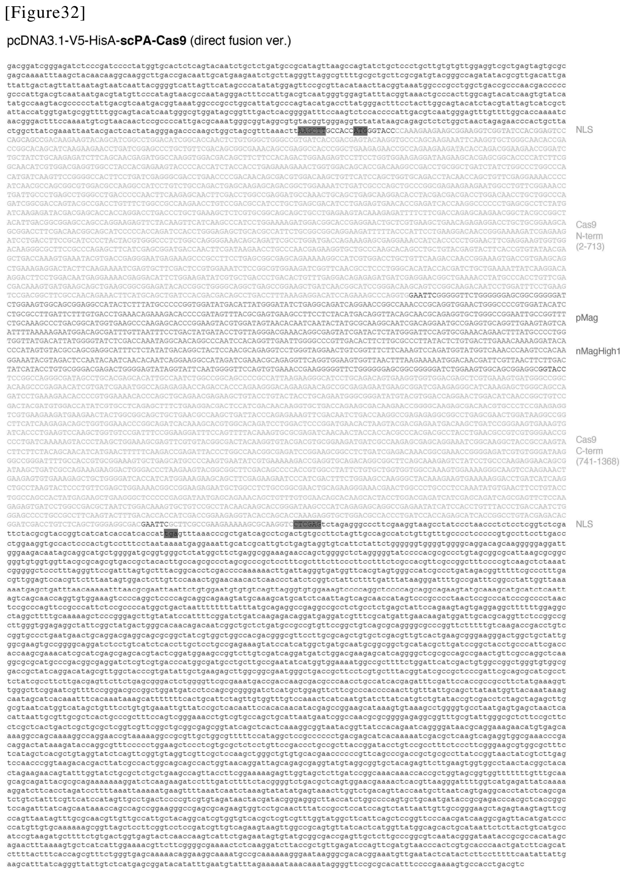

[Figure 32] Figure 32 shows the nucleotide sequence (SEQ ID No: 22) of pcDNA3.1-V5-HisA-scPA-Cas9 (direct binding).

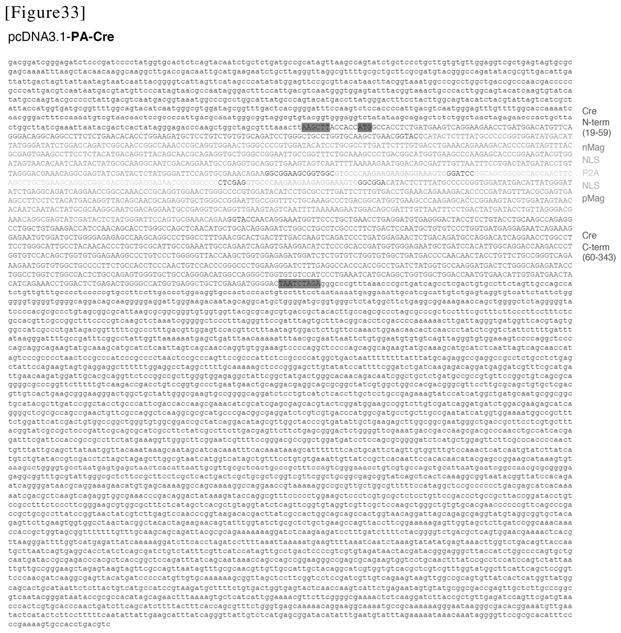

[Figure 33] Figure 33 shows the nucleotide sequence (SEQ ID No: 23) of pcDNA3.1-PA-Cre.

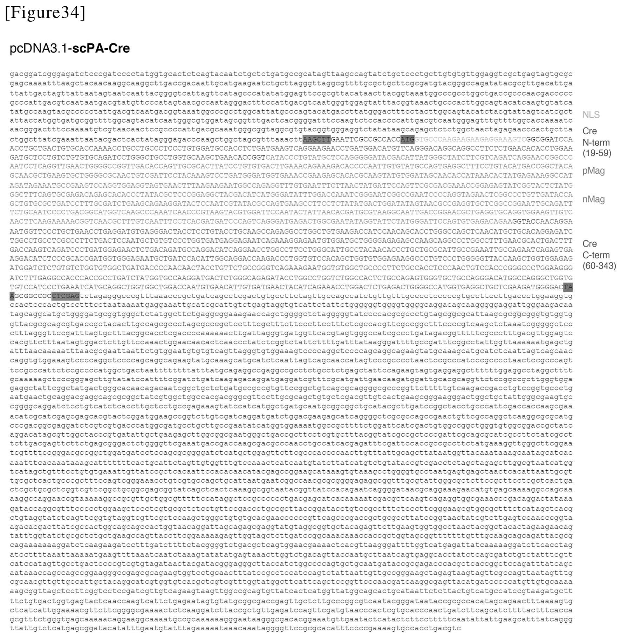

[Figure 34] Figure 34 shows the nucleotide sequence (SEQ ID No: 24) of pcDNA3.1-scPA-Cre.

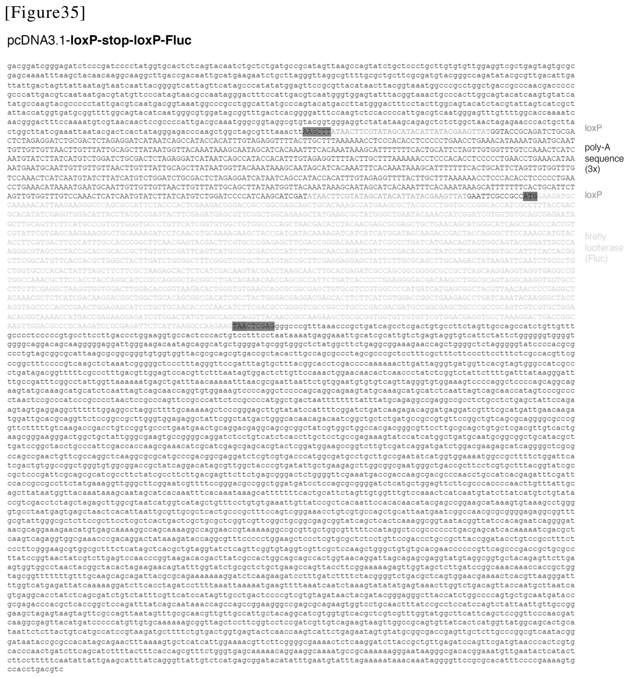

[Figure 35] Figure 35 shows the nucleotide sequence (SEQ ID No: 25) of pcDNA3.1-loxP-stop-loxP-Fluc.

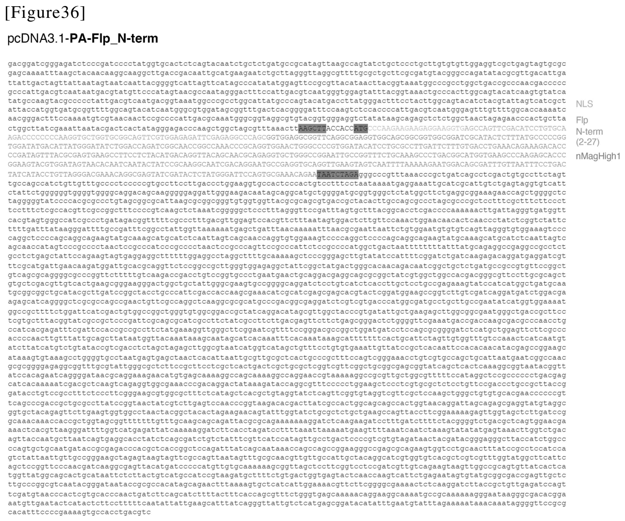

[Figure 36] Figure 36 shows the nucleotide sequence (SEQ ID No: 26) of pcDNA3.1-PA-Flp_N-term.

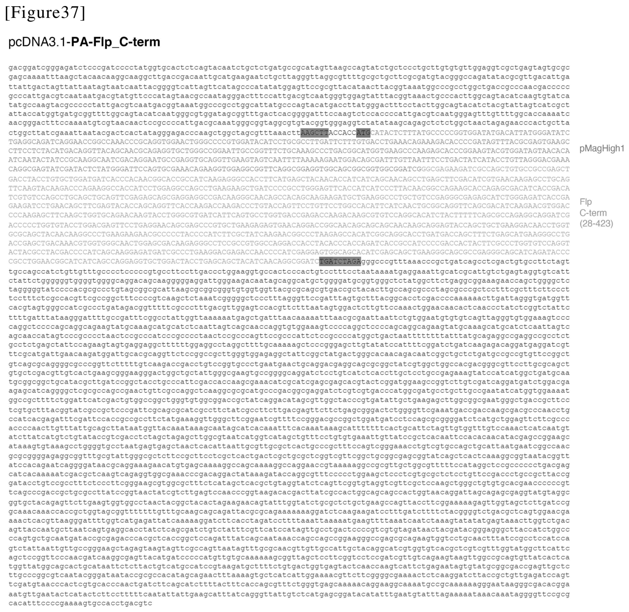

[Figure 37] Figure 37 shows the nucleotide sequence (SEQ ID No: 27) of pcDNA3.1-PA-Flp_C-term.

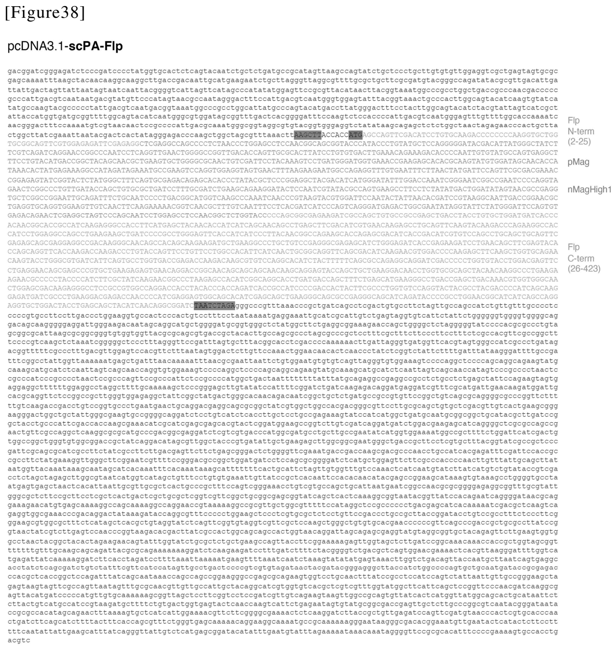

[Figure 38] Figure 38 shows the nucleotide sequence (SEQ ID No: 28) of pcDNA3.1-scPA-Flp.

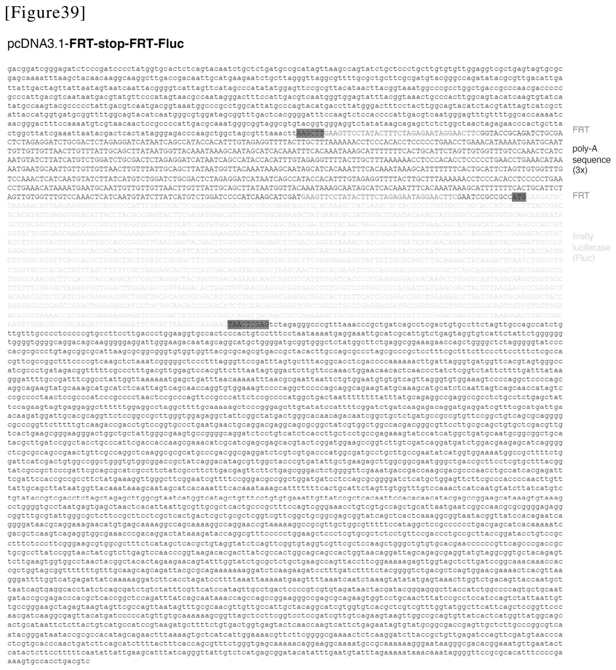

[Figure 39] Figure 39 shows the nucleotide sequence (SEQ ID No: 29) of pcDNA3.1-FRT-stop-FRT-Fluc.

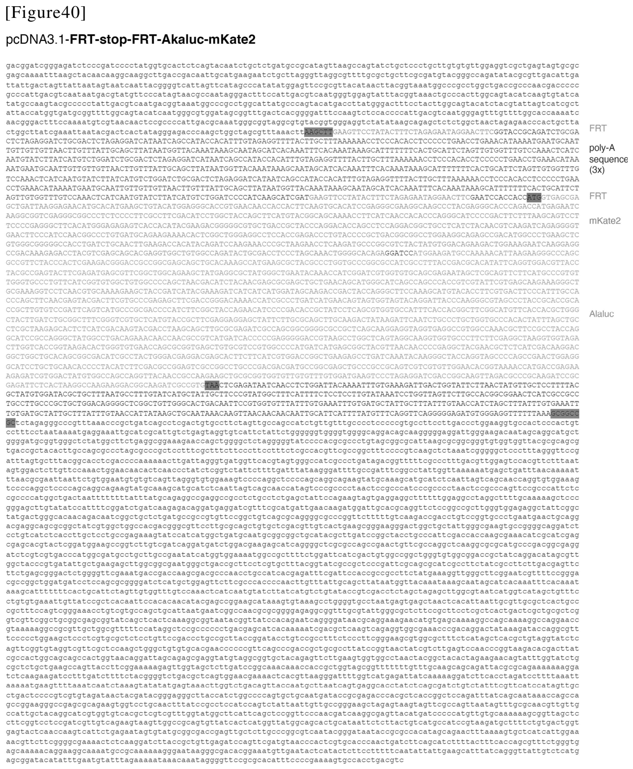

[Figure 40] Figure 40 shows the nucleotide sequence (SEQ ID No: 30) of pcDNA3.1-FRT-stop-FRT-Akaluc-mKate2.

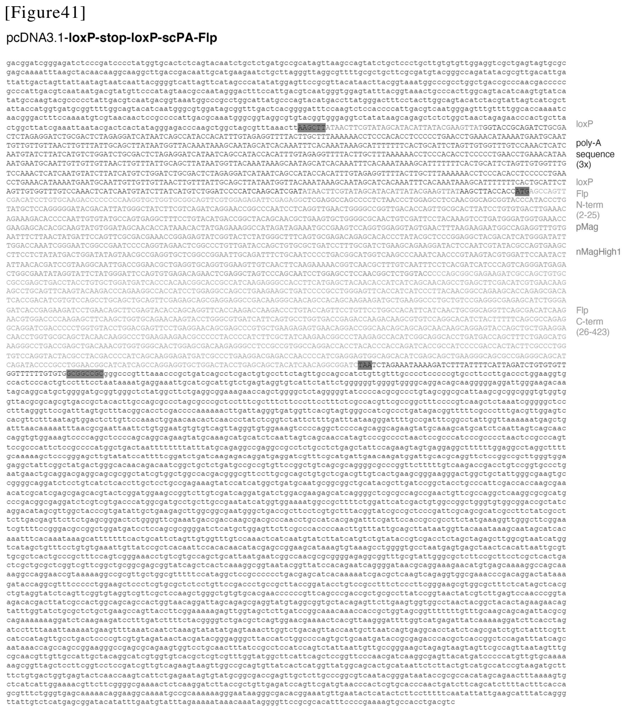

[Figure 41] Figure 41 shows the nucleotide sequence (SEQ ID No: 31) of pcDNA3.1-loxP-stop-loxP-scPA-Flp.

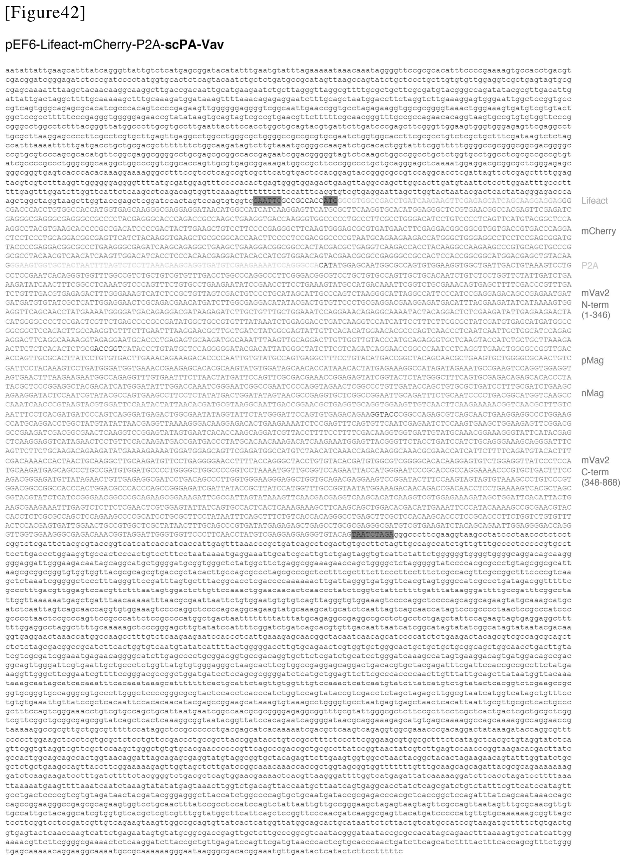

[Figure 42] Figure 42 shows the nucleotide sequence (SEQ ID No: 32) of pEF6-Lifeact-mCherry-P2A-scPA-Vav.

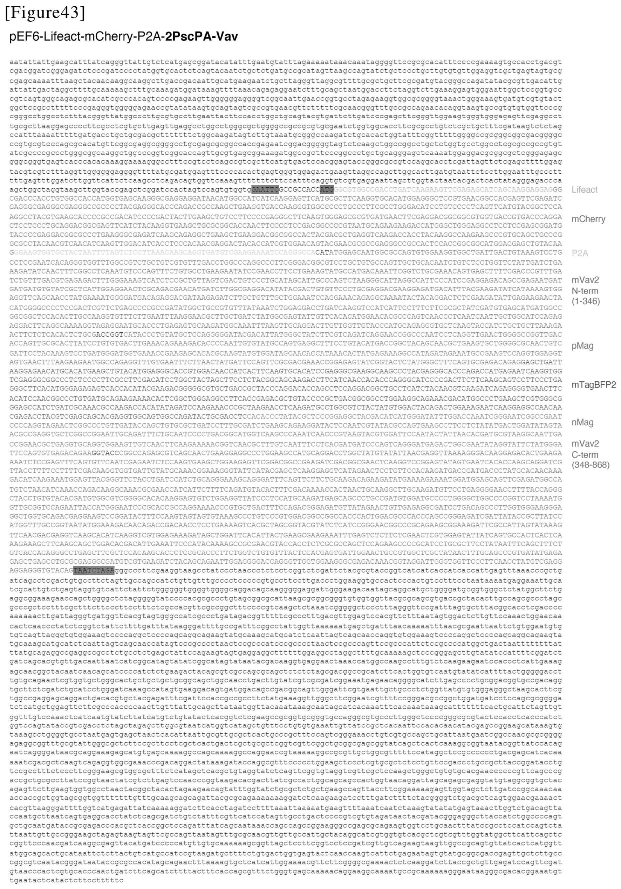

[Figure 43] Figure 43 shows the nucleotide sequence (SEQ ID No: 33) of pEF6-Lifeact-mCherry-P2A-2PscPA-Vav.

### Description of Embodiments

Hereinafter, the embodiments for carrying out the present invention will be described. It is to be noted that, unless otherwise specified, when the term "the present embodiment" is used, it refers to all embodiments described in the present description.

A first embodiment relates to a photoswitching protein consisting of a single polypeptide, differing from the conventional photoswitching protein consisting of two different proteins. The "photoswitching protein" according to the present embodiment is a protein, in which two proteins selected from VVD or VVD mutants (hereinafter, unless otherwise specified, when a "VVD protein" is mentioned, it means VVD or a VVD mutant) are directly linked to each other or indirectly linked via a linker of one amino acid residue or peptide.

In some cases, in the present VVD protein, one or multiple consecutive amino acid residues may be deleted from the terminal amino acid residue on the side linked to another VVD protein towards the opposite terminal side. For example, when the photoswitching protein according to the present embodiment comprises a wild-type VVD and a VVD mutant (referred to as VVD1) linked by a polypeptide linker L (Nter-VVD-L-VVD1-Cter), one or multiple consecutive amino acid residues may be deleted from the C-terminal residue of the wild-type VVD towards the N-terminal side, and/or one or multiple consecutive amino acid residues may be deleted from the N-terminal residue of VVD1 towards the C-terminal side. Herein, in the deletion of "one or multiple" amino acid residues, the term "multiple" means, for example, 2 to 30, preferably 2 to 20, and more preferably 2 to 10. In the photoswitching protein according to the present embodiment, the leak activity of the present photoswitching protein in the dark can be reduced by deleting the amino acid residues in the terminal region of the VVD protein linked to the linker, as described above.

When two VVD proteins that constitute the photoswitching protein according to the present embodiment are indirectly linked, the two VVD proteins may be linked via a linker that is one amino acid residue or a peptide. When the linker is a peptide, the linker may be an oligopeptide (e.g., about 10 or less amino acids), a polypeptide (e.g., about 10 to 100, about 10 to 50, or about 10 to 30 amino acids), a protein, or the like. When the linker is an oligopeptide or a polypeptide, wherein the peptide does not adapt a specific structure, the amino acid that constitutes the linker is not particularly limited, and examples thereof may include glycine (G), serine (S), alanine (A), and threonine (T). In addition, when the linker is a protein, examples of the protein may include, but is not particularly limited to, fluorescent proteins (e.g., BFP, TagBFP, mTagBFP2, mBlueberry2, mCerulean, mTurquoise, ECFP, TagCFP, Rosmarius, meleCFP, mTFP1, KCY, meffCFP, GFP, YFP, Venus, mCherry, iRFP, etc.) and bioluminescent proteins (e.g., NanoLuc, Gaussia luciferase, Renilla luciferase, firefly luciferase, etc.).

The photoswitching protein according to the present embodiment has an activity, by which when the present photoswitching protein is irradiated with a light, the structure thereof is largely changed and the VVD proteins present on both sides of the linker bind to each other, and the VVD proteins are dissociated from each other in the dark (under the circumstances where the present photoswitching protein is not irradiated with a light). If each of two interacting proteins have been linked to each of the two VVD proteins that constitute the photoswitching protein according to the present embodiment (i.e., the N-terminal side and C-terminal side of the photoswitching protein), the photoswitching protein can bring the two proteins close together and into a state where they can interact with each other by being irradiated with a light, and can dissociate the two split fragments by terminating the light irradiation. Herein, the "two interacting proteins" may be two protein fragments that are formed by splitting a single original protein. Therefore, if split fragments of a protein are allowed to each bind to, for example, the N-terminal side and C-terminal side of the photoswitching protein according to the present embodiment, the rebinding and dissociation of the split fragments can be controlled by turning a light ON/OFF in light irradiation, and the activity of the present protein (the protein before splitting) can be controlled.

As mentioned above, the VVD protein in the present embodiment includes wild-type VVD (SEQ ID No: 1) and its mutants. In addition to the VVD mutants described below, it may also be a VVD mutant (SEQ ID No: 36) in which the amino acid residues at positions 1 to 36 of wild-type VVD consisting of the amino acid sequence as set forth in SEQ ID No: 1 are deleted. It has been reported that if the 36 amino acid residues on the N-terminal side of VVD are deleted when the VVD is allowed to express, the expression level thereof is improved (Zoltowski et al., Biochemistry 47: 7012-7019, 2008). Therefore, as such a VVD protein in the present embodiment, a protein, in which the amino acid mutations described below are appropriately introduced into the VVD protein consisting of the amino acid sequence as set forth in SEQ ID No: 1 or SEQ ID No: 36, may also be used.

The photoswitching protein according to the present embodiment is more specifically a protein, in which the C-terminus of the following protein (a) is directly or indirectly linked to the N-terminus of the following protein (b):
(a) a protein comprising an amino acid sequence having a sequence identity of 80% or more to an amino acid sequence comprising a deletion of an X number of consecutive amino acid residues from the C-terminal residue to the N-terminal side of the amino acid sequence as set forth in SEQ ID No: 1 or SEQ ID No: 36, wherein X represents an integer of 0 or greater and 6 or smaller; and
(b) a protein comprising an amino acid sequence having a sequence identity of 80% or more to an amino acid sequence comprising a deletion of a Y number of consecutive amino acid residues from the N-terminal residue to the C-terminal side of the amino acid sequence as set forth in SEQ ID No: 1 or SEQ ID No: 36, wherein Y represents an integer of 0 or greater and 30 or smaller.

Herein, the protein consisting of the amino acid sequence as set forth in SEQ ID No: 1 as set forth below is Vivid (VVD) derived from Neurospora crassa, and the protein consisting of the amino acid sequence as set forth in SEQ ID No: 36 is a VVD mutant consisting of an amino acid sequence comprising a deletion of the amino acids at positions 1 to 36 in the amino acid sequence as set forth in SEQ ID No: 1.

SEQ ID No: 1;
MSHTVNSSTMNPWEVEAYQQYHYDPRTAPTANPLFFHTLYAPGGYDIMGY
LIQIMNRPNPQVELGPVDTSCALILCDLKQKDTPIVYASEAFLYMTGYSNA
EVLGRNCRFLQSPDGMVKPKSTRKYVDSNTINTMRKAIDRNAEVQVEVV
NFKKNGQRFVNFLTMIPVRDETGEYRYSMGFQCETE

SEQ ID No: 36;
HTLYAPGGYDIMGYLIQIMNRPNPQVELGPVDTSCALILCDLKQKDTPIVY
ASEAFLYMTGYSNAEVLGRNCRFLQSPDGMVKPKSTRKYVDSNTINTMRK
AIDRNAEVQVEVVNFKKNGQRFVNFLTMIPVRDETGEYRYSMGFQCETE

In "an amino acid sequence comprising a deletion of an X number of consecutive amino acid residues from the C-terminal residue to the N-terminal side of the amino acid sequence as set forth in SEQ ID No: 1 or SEQ ID No: 36," when X is 0, for example, the concerned amino acid sequence is a sequence comprising no deletion of amino acids in the amino acid sequence as set forth in SEQ ID No: 1 or SEQ ID No: 36. Moreover, when X is 20, the concerned amino acid sequence is an amino acid sequence comprising a deletion of 20 consecutive amino acid residues from the C-terminal residue to the N-terminal side of the amino acid sequence as set forth in SEQ ID No: 1 or SEQ ID No: 36. More specifically, for example, "an amino acid sequence comprising a deletion of 20 consecutive amino acid residues from the C-terminal residue to the N-terminal side of the amino acid sequence as set forth in SEQ ID No: 1" is an amino acid sequence consisting of consecutive amino acid residues from position 1 to position 166 on the amino acid sequence as set forth in SEQ ID No: 1. Herein, X is an integer of 0 or greater and 30 or smaller, preferably an integer of 0 or greater and 20 or smaller, more preferably an integer of 0 or greater and 15 or smaller, and further preferably an integer of 0 or greater and 6 or smaller.

In "an amino acid sequence comprising a deletion of a Y number of consecutive amino acid residues from the N-terminal residue to the C-terminal side of the amino acid sequence as set forth in SEQ ID No: 1 or SEQ ID No: 36," when Y is 0, for example, the concerned amino acid sequence is a sequence comprising no deletion of amino acids in the amino acid sequence as set forth in SEQ ID No: 1 or SEQ ID No: 36. Moreover, when Y is 20, the concerned amino acid sequence is an amino acid sequence comprising a deletion of 20 consecutive amino acid residues from the N-terminal residue to the C-terminal side of the amino acid sequence as set forth in SEQ ID No: 1 or SEQ ID No: 36. More specifically, for example, "an amino acid sequence comprising a deletion of 20 consecutive amino acid residues from the N-terminal residue to the C-terminal side of the amino acid sequence as set forth in SEQ ID No: 1" is an amino acid sequence consisting of consecutive amino acid residues from position 21 to position 186 on the amino acid sequence as set forth in SEQ ID No: 1. Herein, Y is an integer of 0 or greater and 70 or smaller, preferably an integer of 0 or greater and 60 or smaller, more preferably an integer of 0 or greater and 55 or smaller, and further preferably an integer of 0 or greater and 35 or smaller.

In the above-described protein (a) and protein (b) that constitute the photoswitching protein according to the present embodiment, when X in the protein (a) is 0, Y in the protein (b) may be an integer of 1 or greater and 30 or smaller. When Y in the protein (b) is 0, X in the protein (a) may be an integer of 1 or greater and 30 or smaller. That is to say, 1 or greater and 30 or smaller amino acid residues may be deleted at the linking terminus of at least one of the above-described protein (a) and protein (b) that constitute the photoswitching protein according to the present embodiment (i.e., at the terminus on the other VVD protein side or on the side to which the linker binds)

Furthermore, in the present embodiment, the "protein having a sequence identity of 80% or more" means a protein having a sequence identity of 80% or more, 81% or more, 82% or more, 83% or more, 84% or more, 85% or more, 86% or more, 87% or more, 88% or more, 89% or more, 90% or more, 91% or more, 92% or more, 93% or more, 94% or more, 95% or more, 96% or more, 97% or more, 98% or more, or 99% or more, or a protein having a sequence identity of 100%, to the "amino acid sequence comprising a deletion of an X number of consecutive amino acid residues from the C-terminal residue to the N-terminal side of the amino acid sequence as set forth in SEQ ID No: 1 or SEQ ID No: 36," or to the "amino acid sequence comprising a deletion of a Y number of consecutive amino acid residues from the N-terminal residue to the C-terminal side of the amino acid sequence as set forth in SEQ ID No: 1 or SEQ ID No: 36." Herein, except for the protein having a sequence identity of 100%, the "proteins having a sequence identity of 80% or more" are mutants of the protein consisting of the amino acid sequence as set forth in SEQ ID No: 1 or SEQ ID No: 36. The VVD mutant constituting the photoswitching protein according to the present embodiment is not particularly limited, as long as it has properties, by which one VVD protein binds to the other VVD protein constituting the photoswitching protein according to the present embodiment by light irradiation, and in the dark, the bond thereof is canceled (dissociated). For example, VVD mutants described below are desirable.

The above-described desirable mutants are, for example, the mutants disclosed in Patent Literature 1. When the photoswitching protein of the present embodiment, namely, the above-described proteins (a) and (b) are both VVD mutants, it is desirable that at least one amino acid located on the binding surface of one VVD mutant (referred to as "VVD mutant 1"), which binds to another VVD mutant (referred to as "VVD mutant 2"), is substituted from an amino acid contained in wild-type VVD to an amino acid having a positive charge on the side chain, and that at least one amino acid located on the binding surface of VVD mutant 2, which binds to VVD mutant 1, is substituted with an amino acid having a positive charge on the side chain. Such VVD mutants have high binding affinity with each other.

It is to be noted that, in the present description, the term "amino acid" is used in the broadest sense, and includes not only natural amino acids, but also their derivatives and artificial amino acids. Examples of the amino acids in the present description may include, for example, natural amino acids, as well as unnatural amino acids. In addition, examples of the "amino acid" may also include amino acids whose main chain structure differs from that of natural amino acids, amino acids whose side chain structure differs from that of natural amino acids, amino acids having extra methylene in the side chain thereof, and amino acids in which the carboxylic acid functional group amino acid in the side chain thereof is substituted with a sulfonic acid group.

The above-described amino acid having a positive charge in the side chain may be either natural amino acid or unnatural amino acid. When the amino acid having a positive charge in the side chain is natural amino acid, examples thereof may include lysine, arginine, and histidine. Also, the amino acid having a negative charge in the side chain may be either natural amino acid or unnatural amino acid. When the amino acid having a negative charge in the side chain is natural amino acid, examples thereof may include aspartic acid and glutamic acid.

In at least one of the VVD mutants that constitute the photoswitching protein according to the present embodiment, at least one amino acid located in a domain binding to the cofactor, flavin adenine dinucleotide (FAD) (FAD-binding domain), may be substituted. The FAD-binding domain can be appropriately determined by those skilled in the art through protein crystallography, etc. The number of amino acids in the FAD-binding domain to be substituted may be any number that is one or more. The number of substituted amino acids is not particularly limited, and it may be, for example, 1, 2, 3, 4, 5, 6, 7, 8, or 9. In addition, the amino acids in the FAD-binding domain may be substituted with any amino acids, as long as the binding and dissociation between the VVDs or VVD mutants constituting the photoswitching protein are carried out at the desired rate.

The VVD mutant according to the present embodiment will be described in more detail below.

In the present description, in a case where the position of the amino acid sequence of a VVD mutant is specified, the "amino acid corresponding to position Z position on the amino acid sequence as set forth in SEQ ID No. 1 or SEQ ID No. 36" refers to the amino acid on the amino acid sequence of the VVD mutant that corresponds to the position Z on the amino acid sequence of SEQ ID No: 1 or SEQ ID No: 36, when the amino acid sequence as set forth in SEQ ID No: 1 or SEQ ID No: 36 and the amino acid sequences of the VVD mutant are aligned such that their identity becomes maximum.

When the photoswitching protein according to the present embodiment comprises VVD mutant 1 and VVD mutant 2, for example, in the VVD mutant 1, the amino acid corresponding to position 52 on the amino acid sequence as set forth in SEQ ID No: 1 may be an amino acid having a positive charge in the side chain, and in the VVD mutant 2, the amino acid corresponding to position 52 on the amino acid sequence as set forth in SEQ ID No: 1 may be an amino acid having a negative charge in the side chain. Alternatively, in the VVD mutant 1, the amino acid corresponding to position 16 on the amino acid sequence as set forth in SEQ ID No: 36 may be an amino acid having a positive charge in the side chain, and in the VVD mutant 2, the amino acid corresponding to position 16 on the amino acid sequence as set forth in SEQ ID No: 36 may be an amino acid having a negative charge in the side chain. It is to be noted that when the two VVD proteins used in the present embodiment are a combination of mutants, it may be a combination of a mutant consisting of an amino acid sequence in which a mutation has been introduced into the amino acid sequence as set forth in SEQ ID No. 1 and a mutant consisting of an amino acid sequence in which a mutation has been introduced into the amino acid sequence as set forth in SEQ ID No. 36 (the same applies to the combinations of mutants described below).

When the photoswitching protein according to the present embodiment comprises VVD mutant 1 and VVD mutant 2, for example, in the VVD mutant 1, the amino acid corresponding to position 55 on the amino acid sequence as set forth in SEQ ID No: 1 may be an amino acid having a positive charge in the side chain, and in the VVD mutant 2, the amino acid corresponding to position 55 on the amino acid sequence as set forth in SEQ ID No: 1 may be an amino acid having a negative charge in the side chain. Alternatively, in the VVD mutant 1, the amino acid corresponding to position 19 on the amino acid sequence as set forth in SEQ ID No: 36 may be an amino acid having a positive charge in the side chain, and in the VVD mutant 2, the amino acid corresponding to position 19 on the amino acid sequence as set forth in SEQ ID No: 36 may be an amino acid having a negative charge in the side chain.

When the photoswitching protein according to the present embodiment comprises VVD mutant 1 and VVD mutant 2, for example, in the VVD mutant 1, the amino acids corresponding to position 52 and position 55 on the amino acid sequence as set forth in SEQ ID No: 1 may each be arginine, and in the VVD mutant 2, the amino acid corresponding to position 52 on the amino acid sequence as set forth in SEQ ID No: 1 may be aspartic acid, and the amino acid corresponding to position 55 thereon may be glycine. Alternatively, in the VVD mutant 1, the amino acids corresponding to position 16 and position 19 on the amino acid sequence as set forth in SEQ ID No: 36 may each be arginine, and in the VVD mutant 2, the amino acid corresponding to position 16 on the amino acid sequence as set forth in SEQ ID No: 36 may be aspartic acid, and the amino acid corresponding to position 19 thereon may be glycine.

When the photoswitching protein according to the present embodiment comprises VVD mutant 1 and VVD mutant 2, in at least one of the mutants, the amino acid located in the FAD-binding domain may be an amino acid different from a wild-type amino acid.

For example, in at least any one of the VVD mutant 1 or the VVD mutant 2, the amino acids corresponding to position 74 and/or position 85 on the amino acid sequence as set forth in SEQ ID No: 1 may be amino acids different from the amino acids in the amino acid sequence as set forth in SEQ ID No: 1. Otherwise, in at least any one of the VVD mutant 1 or the VVD mutant 2, the amino acids corresponding to position 135 and/or position 165 on the amino acid sequence as set forth in SEQ ID No: 1 may be amino acids different from the amino acids in the amino acid sequence as set forth in SEQ ID No: 1.

Alternatively, in at least any one of the VVD mutant 1 or the VVD mutant 2, the amino acids corresponding to position 38 and/or position 49 on the amino acid sequence as set forth in SEQ ID No: 36 may be amino acids different from the amino acids in the amino acid sequence as set forth in SEQ ID No: 36. Otherwise, in at least any one of the VVD mutant 1 or the VVD mutant 2, the amino acids corresponding to position 99 and/or position 129 on the amino acid sequence as set forth in SEQ ID No: 36 may be amino acids different from the amino acids in the amino acid sequence as set forth in SEQ ID No: 36.

More specifically, when the photoswitching protein according to the present embodiment comprises VVD mutant 1 and VVD mutant 2, for example, in the VVD mutant 1 and the VVD mutant 2, the amino acid corresponding to position 85 on the amino acid sequence as set forth in SEQ ID No: 1 may be valine, or the amino acids corresponding to position 74 and position 85 on the amino acid sequence as set forth in SEQ ID No: 1 may each be valine. Otherwise, when the photoswitching protein according to the present embodiment comprises VVD mutant 1 and VVD mutant 2, for example, in the VVD mutant 1 and the VVD mutant 2, the amino acids corresponding to position 135 and position 165 on the amino acid sequence as set forth in SEQ ID No: 1 may each be isoleucine.

Alternatively, when the photoswitching protein according to the present embodiment comprises VVD mutant 1 and VVD mutant 2, for example, in the VVD mutant 1 and the VVD mutant 2, the amino acid corresponding to position 49 on the amino acid sequence as set forth in SEQ ID No: 36 may be valine, or the amino acids corresponding to position 38 and position 49 on the amino acid sequence as set forth in SEQ ID No: 1 may each be valine. Otherwise, when the photoswitching protein according to the present embodiment comprises VVD mutant 1 and VVD mutant 2, for example, in the VVD mutant 1 and the VVD mutant 2, the amino acids corresponding to position 99 and position 129 on the amino acid sequence as set forth in SEQ ID No: 36 may each be isoleucine.

The two VVD proteins that constitute the photoswitching protein according to the present embodiment may each be bound to or fused with "other substances (i.e., substances different from individual proteins that constitute the photoswitching proteins)." Such "other substances" are not particularly limited, and may be any substances. Examples of such other substances may include various biological materials such as proteins, lipids, sugars, and nucleic acids. The photoswitching proteins according to the present embodiment and "other substances" may be bound to one another via linkers or the like, and when such "other substances" are proteins, they may be fused with one another. More specifically, examples of such "other substances" may include interacting "protein A" and "protein B," such as Cas9 Nter" and "Cas9 Cter" shown in Figure 2c, "CreN" and "CreC" shown in Figure 7a, "FlpN" and "FlpC" shown in Figure 7c, and "mVav2 N-ter" and "mVav2 C-ter" shown in Figure 22a. In this case, when the photoswitching protein according to the present embodiment is irradiated with a blue light (the wavelength range is not particularly limited, and it is, for example, approximately 400 nm to 500 nm), the interaction between Protein A and Protein B can be induced, and when the blue light irradiation is terminated, Protein A and Protein B can be dissociated from each other. Therefore, by using the photoswitching proteins according to the present embodiment, it is possible to freely control the interaction between proteins by turning light irradiation ON/OFF. In addition to the examples shown herein, there are other useful intended uses of the photoswitching proteins according to the present embodiment (see Non Patent Literature 1 and Non Patent Literature 2, etc.).

A second embodiment relates to a nucleic acid (DNA or RNA) that encodes the photoswitching protein according to the present embodiment.

The nucleic acid according to the present embodiment can be prepared by a method known in the present technical field, or a method obtained by appropriately modifying the method. For example, a desired nucleic acid may be obtained by introducing a desired mutation into DNA that encodes the wild-type VVD. Alternatively, the present nucleic acid can also be synthesized by an automatic synthesizer. When individual proteins that constitute the photoswitching protein according to the present embodiment is fused with other proteins, the nucleic acids that encode such fusion proteins are also included in the nucleic acid according to the present embodiment.

The photoswitching protein according to the present embodiment can be prepared by incorporating a nucleic acid encoding it into a suitable expression vector, then transforming or transfecting suitable host cells with the expression vector, then culturing the host cells in an appropriate medium, and then purifying each expressed protein.

Examples of the host cells for protein expression that may be used herein may include bacterial cells (e.g., Escherichia coli B strain, E. coli K12 strain, Corynebacterium ammoniagenes, C. glutamicum, Serratia liquefaciens, Streptomyces lividans, Pseudomonas putida, etc.), molds (e.g., Penicillium camembertii, Acremonium chrysogenum, etc.), animal cells, plant cells, baculovirus/insect cells, and yeast cells (e.g., Saccharomyces cerevisiae and Pichia pastoris, etc.).

As an expression vector for protein expression, a vector suitable for various types of host cells can be used. Examples of the expression vector that can be used herein may include: pBR322, pBR325, pUC118, pET, etc. (for E. coli hosts); pEGFP-C, pEGFP-N, etc. (for animal cell hosts); pVL1392, pVL1393, etc. (for insect cell hosts; baculovirus vectors); and pG-1, Yep13, pPICZ, etc. (for yeast cell hosts). These expression vectors may comprise a replication origin, a selection marker, and a promoter, which are suitable for each of these vectors, and the vectors may also comprise, if necessary, an enhancer, a transcription termination sequence (terminator), a ribosome-binding site, a polyadenylation signal, and the like. Furthermore, in order to facilitate purification of the expressed polypeptide, a nucleotide sequence for fusing and expressing a FLAG tag, a His tag, an HA tag, a GST tag, etc. may be inserted into the expression vector.

The expression vector can be prepared by a method known to those skilled in the art, and can also be prepared using a commercially available kit, etc., as appropriate.

When the expressed protein is extracted from cultured cell masses or cultured cells, the cell masses or the cultured cells are collected by a known method after completion of the culture, and the collected cell masses or cells are then suspended in a suitable buffer. Thereafter, the suspension is subjected to ultrasonic wave, lysozyme and/or freezing-thawing, etc., so that the cell masses or cells are disintegrated. Thereafter, the resultant is subjected to centrifugation or filtration to obtain a soluble extract. In particular, when cultured cells are used as hosts, it is desirable to obtain the protein expressed in a culture supernatant by recovering the supernatant. An appropriate combination of known separation and/or purification methods is applied to the obtained extract or culture supernatant, so that a protein of interest can be obtained. Examples of the known separation and/or purification methods that is used herein may include: methods of utilizing solubility, such as salting-out or a solvent precipitation method; methods of mainly utilizing a difference in molecular weights, such as a dialysis method, an ultrafiltration method, a gel filtration method, or SDS-PAGE; methods of utilizing a difference in electric charges, such as ion exchange chromatography; methods of utilizing specific affinity, such as affinity chromatography (for example, methods, in which when a polypeptide is expressed together with a GST tag, a glutathione-bound carrier resin is used, when a polypeptide is expressed together with a His tag, a Ni-NTA resin or a Co-based resin is used, when a polypeptide is expressed together with a HA tag, an anti-HA antibody resin is used, and when a polypeptide is expressed together with a FLAG tag, an anti-FLAG antibody-bound resin or the like is used); methods of utilizing a difference in hydrophobicity, such as reverse phase high performance liquid chromatography; and methods of utilizing a difference in isoelectric points, such as an isoelectric focusing method.

A third embodiment relates to the photoswitching protein according to the first embodiment, in which two proteins selected from a VVD protein group are linked to each other via a fluorescent protein or a bioluminescent protein. In some cases, one or multiple amino acid residues at the terminus of the present VVD protein that binds to the fluorescent protein or bioluminescent protein may be deleted (see the description regarding the first embodiment).

In the photoswitching protein according to the third embodiment, a fluorescent protein or a bioluminescent protein is present between the two VVD proteins. The fluorescent protein or the bioluminescent protein may directly bind to the VVD proteins, or may indirectly bind to the VVD proteins via a linker (for example, a peptide, etc.) or the like.

In a case where a fluorescent protein is present between two VVD proteins, when the fluorescent protein is excited with a near-infrared light that penetrates biological tissues (e.g., by two-photon excitation), if the excitation energy emitted by the fluorescent protein is acceptable to the VVD proteins, FRET (Fluorescence Resonance Energy Transfer) occurs from the fluorescent protein to the VVD proteins, so that it can induce the binding between the VVD proteins. When a fluorescent protein is excited with a near-infrared light, a two-photon excitation method can be used. According to the two-photon excitation method, two photons with half the energy of one-photon excitation, i.e., two photons with twice the wavelength, are simultaneously absorbed by the fluorescent protein, thereby generating fluorescence with the same wavelength as that by one-photon excitation. For example, a fluorescent protein that is excited with a wavelength of 400 nm in the one-photon excitation will be excited with a wavelength of 800 nm in the two-photon excitation. In other words, by using a near-infrared light with a wavelength of around 800 nm as an excitation light, it is advantageous in that it becomes less susceptible to the influence of scattering within biological samples (e.g., biological tissues), and in that it becomes possible to observe deep inside. Therefore, the fluorescent protein constituting the photoswitching protein according to the third embodiment is desirably one that is capable of two-photon excitation with a near-infrared light (e.g., a light with approximately 650 nm to 900 nm) and that causes FRET from the fluorescent protein to the VVD proteins. Such a fluorescent protein can be easily selected by those skilled in the art, and examples of the fluorescent protein may include, but are not particularly limited to, BFP, TagBFP, mTagBFP2, mBlueberry2, mCerulean, mTurquoise, ECFP, TagCFP, Rosmarius, meleCFP, mTFP1, KCY, and meffCFP.

Moreover, in a case where a bioluminescent protein is present between two VVD proteins, when the energy generated by an oxidation reaction catalyzed by the bioluminescent protein is acceptable to the VVD proteins in the presence of a luminescent substrate, BRET (Bioluminexcence Resonance Energy Transfer) occurs from the bioluminescent protein to the VVD proteins, so that it can induce the binding between the VVD proteins. Examples of the bioluminescent protein that can be used herein may include NanoLuc, Gaussia luciferase, Renilla luciferase, and firefly luciferase.

A fourth embodiment relates to a method for controlling the distance between two proteins, the method comprising a step of allowing one VVD protein constituting the photoswitching protein according to the present embodiment to bind (including "fuse") to one of the two proteins, a step of allowing the other VVD protein constituting the present photoswitching protein to bind (including "fuse") to the other of the two proteins, and a step of irradiating the photoswitching protein with a light. Herein, the two "proteins" are preferably interacting proteins, and the two proteins may also be two split fragments generated by splitting a single protein, such that the activity of the original protein is restored upon rebinding (reassociation) of the two fragments. The VVD protein and the concerned protein may be bound via a linker, or may also be bound (fused) directly.

The fourth embodiment can also be carried out in cells. Herein, the "cells" include both in vitro cells and in vivo cells. A method of introducing into cells a protein complex composed of the photoswitching protein according to the present embodiment and the "two proteins" according to the fourth embodiment can be selected, as appropriate, by those skilled in the art. For example, a vector expressing the protein complex may be introduced into the cells to express the protein complex within the cells, or the protein complex may be directly introduced into the cells using a cell membrane-permeable peptide or the like. When the protein complex is transferred into the nucleus or intracellular organelles, the protein complex may be introduced into the cells, in a state in which the protein complex is fused with a migration signal peptide (nuclear localization signal peptide, mitochondrial localization signal peptide, etc.).

When the present description is translated into English and the English description includes singular terms with the articles "a," "an," and "the," these terms include not only single items but also multiple items, unless otherwise clearly specified from the context that it is not the case. In addition, in the present description, the terms "equivalent" and "approximately" refer to a numerical range of ±10%.

Hereinafter, the present invention will be further described in the following examples. However, these examples are only illustrative examples of the embodiments of the present invention, and thus, are not intended to limit the scope of the present invention.

### Examples

### 1. Methods

### 1-1. Reagents and materials

The synthesis of all oligonucleotides used in plasmid construction was outsourced to Eurofines Genomics, Integrated DNA Technology, or GenScript. Mammalian cell expression vectors pcDNA3.1(+), pcDNA3.1/V5-His A, and pEF6/V5-His A were purchased from Thermo Fisher Scientific. Plasmid construction was performed by conventional methods based on overlap extension PCR, the cleavage of PCR products with restriction enzymes, and ligation of double-stranded DNA with T4 DNA ligase. As DNA polymerase for PCR reactions, PrimeSTAR HS DNA Polymerase (Takara Bio, Japan) or KOD One PCR Master Mix (TOYOBO) was used according to the attached instructions for use. All restriction enzymes used for DNA cleavage were purchased from Takara Bio or New England BioLabs (NEB), and were used according to the attached instructions for use. As T4 DNA ligase for ligation reactions, Ligation high Ver.2 (TOYOBO) was used according to the attached instructions for use. All of the produced constructs were confirmed using DNA sequencing analysis service (Eurofines Genomics). All plasmid constructs were purified using QIAGEN Plasmid Plus Kits (QIAGEN).

### 1-2. Plasmid Construction

In order to construct the expression plasmid for photoactivable Cas9, sequences encoding the codon-optimized N-terminal side fragment and C-terminal side fragment of Streptococcus pyogenes Cas9 fused with the SV40 T antigen-derived nuclear localization signal (NLS) were amplified from Addgene plasmid (ID: 42230). The synthesis of sequences encoding pMag and nMagHigh1, the codon of which had been optimized for expression in mammalian cells, was outsourced to Eurofines Genomics, Integrated DNA Technology, or GenScript. The nucleotide sequences of the expression constructs for PA-Cas9_N-term, PA-Cas9_C-term, scPA-Cas9 (linked via a flexible linker), scPA-Cas9 (linked via a rigid linker), and scPA-Cas9 (directly fused) were inserted into the HindIII and XhoI sites of the pcDNA3.1/V5-His A vector containing a cytomegalovirus (CMV) promoter. The gRNA expression vectors were constructed by introducing gRNAs targeting human VEGFA, human DNMT1, human EMX1, and mouse K-ras into oligo DNAs annealed to the BbsI site of the pSPgRNA vector (ID: 47108) purchased from Addgene. The target sequences and oligonucleotides used to construct the gRNA expression vectors are shown in Figure 4 and Table 1.

**[Table 1]**

| **target locus name** | **oligonucleotide (for top strand)** | **oligonucleotide (for bottom strand)** |
|---|---|---|
| human *VEGFA* | 5'- CACCGGTGAGTGAGTGTGTGCGTG -3' (SEQ ID No:2) | 5'- AAACCACGCACACACTCACTCACC -3' (SEQ ID No:3) |
| human *DNMT1* | 5'- CACCGCCGTTTCCCTCACTCCTGCT -3' (SEQ ID No:4) | 5'- AAACAGCAGGAGTGAGGGAAACGGC -3' (SEQ ID No:5) |
| human *EMX1* | 5'- CACCGAGTCCGAGCAGAAGAAGAA -3' (SEQ ID No:6) | 5'- AAACTTCTTCTTCTGCTCGGACTC -3' SEQ ID No:7) |
| mouse *K-ras* | 5'- CACCGCTAATTCAGAATCACTTTG -3' (SEQ ID No:8) | 5'- AAACCAAAGTGATTCTGAATTAGC -3' (SEQ ID No:9) |

In order to construct photoactivatable Cre, the synthesis of sequences, in which NLS (nuclear localization signal) was fused to the N-terminal side fragment and C-terminal side fragment of Cre recombinase whose codon had been optimized for expression in mammalian cells, was outsourced to Invitrogen or Thermo Fisher Scientific. The synthesis of sequences encoding pMag (SEQ ID No: 37) and nMag (SEQ ID No: 38), the codon of which had been optimized for expression in mammalian cells, was outsourced to Eurofines Genomics, Integrated DNA Technology, or GenScript. Photoactivatable Cre sequences were constructed by inserting PA-Cre and scPA-Cre into the HindIII-XbaI site or HindIII-XhoI site of the pcDNA3.1(+) vector having a CMV promoter. Regarding construction of luciferase reporters to detect Cre-loxP recombinase activity, polyadenylation "stop" signal (poly(A)) repeat sequences that were adjacent to the loxP site in the same direction as the loxP site were amplified from the Addgene plasmid (ID: 22797). The sequence encoding firefly luciferase (Fluc) was amplified from the pGL4.31 vector (Promega). The sequence of the loxP-stop-loxP-Fluc construct was inserted into the HindIII-XhoI sites of the pcDNA3.1(+) vector having a CMV promoter.

In order to construct photoactivatable Flp, the synthesis of sequences, in which NLS was fused to the N-terminal side fragment and C-terminal side fragment of Flp recombinase whose codon had been optimized for expression in mammalian cells, was outsourced to Eurofines Genomics. The synthesis of sequences encoding pMag, nMag, pMagHigh1, and nMagHigh1, the codon of which had been optimized for expression in mammalian cells, was outsourced to Eurofines Genomics, Integrated DNA Technology, or GenScript. Photoactivatable Flp sequences were constructed by inserting PA-Flp_N-terminal side, PA-Flp_C-terminal side, and scPA-Flp into the HindIII-XbaI site of the pcDNA3.1(+) vector having a CMV promoter. A luciferase reporter construct for detecting Flp-FRT recombinase activity was prepared by substituting each of the two loxP sites of the loxP-stop-loxP-Fluc construct with an FRT site. A photoactivatable Flp construct regarding Cre-dependent recombination cascade was constructed by inserting an scPA-Flp sequence into the HindIII-NotI site of a loxP-stop-loxP-Fluc reporter vector. Regarding a fluorescence reporter for detecting Flp-FRT recombinase activity, the synthesis of a sequence encoding mKate2 fused to Akaluc luciferase was outsourced to Eurofines Genomics, and the synthesized sequence was inserted into the HindIII-NotI of an FRT-stop-FRT-Fluc reporter vector to construct the fluorescence reporter.

In order to construct photoactivatable Vav, the synthesis of sequences encoding Lifeact-mCherry, P2A self-cleaving 2A peptide, mouse Vav2 N-terminal side fragment and C-terminal side fragment, Rho GEF, pMag and nMag, in which the codon had been optimized for expression in mammalian cells, was outsourced to Eurofines Genomics. The synthesized sequences were inserted into the EcoRI-XbaI site of a pEF6/V5-His A vector having a human EF-1α promoter. Regarding construction of photoactivatable Vav by two-photon excitation, the synthesis of sequences encoding pMag, mTagBFP2 and nMag was outsourced to Eurofines Genomics, and the synthesized sequences were then inserted into the AgeI-KpnI site of the scPA-Vav construct.

The nucleotide sequences of all the produced constructs are shown in Figures 28 to 43.

### 1-2 Cell culture

HEK293T cells, COS-7 cells and NIH3T3 cells (American Type Culture Collection (ATCC)), and HeLa cells (Japanese Collection of Research Bioresources (JCRB) cell bank), were cultured in a DMEM medium (Dulbecco's Modified Eagle Medium) (Thermo Fisher Scientific), which was supplemented with 0% fetal bovine serum (Thermo Fisher Scientific), 100 units/mL penicillin and 100 µg/mL streptomycin (Thermo Fisher Scientific), and 2 mM L-glutamine or 2 mM GlutaMAX (Thermo Fisher Scientific), at 37°C under conditions of 5% CO₂.

A 5-11 cell line derived from the pancreatic cancer of LSL-KrasG12D/+; LSL-Trp53R172H/+; ptfla-Cre (KPC) mice was established by the previously reported method (Fuchigami et al., Scientific Reports 8:14211 (2018)). The 5-11 cells were cultured in an RPMI-1640 medium (Roswell Park Memorial Institute 1640 Medium) (Thermo Fisher Scientific) , which was supplemented with 20% FBS and 1×Antibiotic-Antimycotic Mixed Solution (Nacalai Tesque), at 37°C under conditions of 5% CO₂.

### 1-3 Light source

Regarding a blue light to be irradiated in the assays of cultured cells and in vivo mouse experiments, LED light source (70 ± 20 nm; CCS) was used.

### 1-4. Genome editing experiments

Regarding an indel mutation experiment mediated by non-homologous end joining (NHEJ) using HEK293K cells, the cells were seeded at 100,000 cells/well in a 24-well plate (Thermo Fisher Scientific) and were then cultured at 37°C under conditions of 5% CO₂ for 24 hours. Thereafter, the cells were transfected using Lipofectamine 3000 (Thermo Fisher Scientific) according to the attached instructions for use. The NLS-N-terminal side fragment of Cas9-pMag, the nMagHigh1-C-terminal side fragment of Cas9-NLS, and the cDNA encoding gRNA were set to at a ratio of 1 : 1 : 1. The total amount of the cDNA was set to 0.2741 µg/well. As a positive control and a negative control, cDNA encoding full-length Cas9 or pcDNA3.1/V5-His A (empty vector) was transfected at a ratio of 1 : 1. Twenty hours after the transfection, the cells were cultured in the dark or under blue light irradiation (1.0 W/m⁻²) for 24 hours. Thereafter, genomic DNA was recovered using Blood Cultured Cell Genomic DNA Extraction Mini Kit (Favorgen) according to the attached instructions for use.

Regarding an indel mutation experiment mediated by non-homologous end joining (NHEJ) using HeLa cells, the cells were seeded at 100,000 cells/well in a 24-well plate (Thermo Fisher Scientific) and were then cultured at 37°C under conditions of 5% CO₂ for 96 hours. Thereafter, the cells were transfected using X-tremeGENE 9 reagent (Thermo Fisher Scientific) according to the attached instructions for use. The NLS-N-terminal side fragment of Cas9-pMag, the nMagHigh1-C-terminal side fragment of Cas9-NLS, and the cDNA encoding gRNA were set to at a ratio of 1 : 1 : 1. The cDNAs encoding scPA-Cas9 and gRNA were set to at a ratio of 1 : 1. The total amount of the cDNA was set to 0.1 µg/well. As a positive control and a negative control, cDNA encoding full-length Cas9 or pcDNA3.1/V5-His A (empty vector) was transfected at a ratio of 1 : 1. Immediately after the transfection, the cells were cultured in the dark or under blue light irradiation (1.0 W/m⁻²) for 48 hours. Thereafter, genomic DNA was recovered using QuickExtract DNA Solution kit (Lucigen) according to the attached instructions for use.

Regarding an indel mutation experiment mediated by non-homologous end joining (NHEJ) using 5-11 cells, the cells were seeded at 100,000 cells/well in a 10-cm culture dish and were then cultured at 37°C under conditions of 5% CO₂ for 24 hours. Subsequently, 400,000 cells per cuvette were subjected to gene transfer by a nucleofection method, using SG cell line 4D-Nucleofector X kit S (Lonza) and EN-158 program according to the attached instructions for use. The NLS-N-terminal side fragment of Cas9-pMag, the nMagHigh1-C-terminal side fragment of Cas9-NLS, and the cDNA encoding gRNA were set to at a ratio of 1 : 1 : 1. The cDNAs encoding scPA-Cas9 and gRNA were subjected to nucleofection at a ratio of 2 : 1. The total amount of the cDNA was set to 2.0 µg/well. As a positive control and a negative control, cDNA encoding full-length Cas9 or pcDNA3.1/V5-His A (empty vector) was subjected to nucleofection at a ratio of 2 : 1. After the nucleofection, the cells were incubated in a nucleocuvette at room temperature for 10 minutes. Thereafter, the cells were suspended in an RPMI-1640 medium that had been warmed in advance. The suspended cells were seeded at 40,000 cells/well in a 96-well plate, and immediately after the seeding, the cells were cultured in the dark or under blue light irradiation (1.0 W/m⁻²) for 48 hours. Thereafter, genomic DNA was recovered.

### 1-5. Genomic PCR

Genomic regions containing nuclease target sites were amplified by a touchdown PCR method using PrimeSTAR HS DNA Polymerase (TaKaRa Bio). The touchdown PCR was performed under the following conditions:
- Initial denaturation: 98°C for 3 minutes
- Touchdown PCR (10 cycles):
   Denaturation (98°C, 10 minutes), annealing (decreasing 1°C for every cycle from 72°C; 30 seconds), and elongation (72°C, 1 minute)
- Ordinary PCR (25 cycles):
   Denaturation (98°C, 10 seconds), annealing (62°C, 30 seconds), and elongation (72°C, 1 minute)
- Final elongation reaction: 72°C, 3 minutes

The sequences of the PCR primers are shown in Table 2. The amplified PCR products were purified using FastGene Gel/PCR Extraction Kits (Nippon genetics, Japan).

**[Table 2]**

| **primer name** | **oligonucleotide sequences** |
|---|---|
| human *VEGFA* forward | 5'- TCCAGATGGCACATTGTCAG -3' (SEQ ID No:10) |
| human *VEGFA* reverse | 5' - AGGGAGCAGGAAAGTGAGGT -3' (SEQ ID No:11) |
| human *DNMT1* forward | 5' - AGCCCGAGAGAGTGCCTCAGGT -3'(SEQ ID No:12) |
| human *DNMT1* reverse | 5'- CCACACATGTGAACGGACAGATTGAC -3' (SEQ ID No:13) |
| human *EMX1* forward | 5' - GGAGCAGCTGGTCAGAGGGG -3' (SEQ ID No:14) |
| human *EMX1* reverse | 5' - GGGAAGGGGGACACTGGGGA -3' (SEQ ID No:5) |
| mouse *K-ras* forward | 5' - GTCGACAAGCTCATGCGGGTG -3' (SEQ ID No:16) |
| mouse *K-ras* reverse | 5'- CAAAATGCAAATACAAAGCACGGATGGC -3' (SEQ ID No:17) |

### 1-6. Sequencing analysis

The PCR products prepared as mentioned above were each subcloned into pcDNA3.1/V5-His A. Plasmids were isolated, and were then subjected to Sanger sequencing. TIDE analysis (https://tide.deskgen.com/) was performed on the purified PCR products.

### 1-7. Site-specific recombination experiments

Regarding luciferase assays, COS-7 cells or NIH3T3 cells were seeded at 10,000 cells/well or 15,000 cells/well in a 96-well transparent, flat-bottom, black-walled microplate (Greiner Bio-One, Austria), and were then cultured at 37°C under conditions of 5% CO₂ for 24 hours. The cells were transfected with cDNA encoding PA-Cre, scPA-Cre, PA-Flp or scPA-Flp and a luciferase reporter (a loxP-stop-loxP Flue reporter or an FRT-stop-FRT Flue reporter) at a ratio of 1 : 20, using Lipofectamine 3000 reagent at 37°C. The total amount of the DNA used was set to 0.1 µg/well. Twenty-four hours after the transfection, the cells were cultured in the dark or under blue light irradiation (1.0 W/m⁻²) for 24 hours. Immediately before the luciferase assay, the medium was exchanged with an HBSS medium (Hanks' balanced salt solution) (Thermo Fisher Scientific) containing 0.2 mM D-luciferin potassium salt (Wako). Bioluminescence was measured at room temperature for 10 seconds per well, using a Centro XS³ LB 960 plate-reading luminometer (Berthold Technologies).

Regarding the control experiments shown in Figure 9 and Figure 11, COS-7 cells were seeded at 10,000 cells/well or 15,000 cells/well in a 96-well transparent, flat-bottom, black-walled microplate, and were then cultured at 37°C under conditions of 5% CO₂ for 24 hours. The cells were transfected with cDNA encoding PA-Cre or scPA-Cre and a loxP-stop-loxP Flue reporter at a ratio of 1 : 10, using X-tremeGENE 9 reagent at 37°C. The total amount of the DNA used was set to 0.05 µg/well. The cells were transfected with cDNA encoding PA-Flp or scPA-Flp and a loxP-stop-loxP Fluc reporter at a ratio of 1 : 10, using Lipofectamine 3000 reagent at 37°C. The total amount of the DNA used was set to 0.1 µg/well. Twenty-four hours after the transfection, the cells were cultured in the dark or under blue light irradiation (1.0 W/m⁻²) for 24 hours. Immediately before the luciferase assay, the medium was exchanged with an HBSS medium containing 0.2 mM D-luciferin potassium salt. Bioluminescence was measured at room temperature for 10 seconds per well, using a Centro XS³ LB 960 plate-reading luminometer.

Regarding the irradiation intensity dependence experiments shown in Figure 9 and Figure 11, COS-7 cells were seeded at 10,000 cells/well or 15,000 cells/well in a 96-well transparent, flat-bottom, black-walled microplate, and were then cultured at 37°C under conditions of 5% CO₂ for 24 hours. The cells were transfected with cDNA encoding scPA-Cre and a loxP-stop-loxP Fluc reporter at a ratio of 1 : 10, using X-tremeGENE 9 reagent at 37°C. The total amount of the DNA used was set to 0.05 µg/well. The cells were transfected with cDNA encoding scPA-Flp and a loxP-stop-loxP Fluc reporter at a ratio of 1 : 10, using Lipofectamine 3000 reagent at 37°C. The total amount of the DNA used was set to 0.1 µg/well. Twenty-four hours after the transfection, the cells were cultured in the dark or under blue light irradiation (0.01, 0.03, 0.1, 0.3, 1.0, 1.6 and 3.3 W/m⁻², or 0.01, 0.03, 0.05, 0.1, 0.3, 1.0, 1.6 and 3.0 W/m⁻²) for 24 hours. Immediately before the luciferase assay, the medium was exchanged with an HBSS medium containing 0.2 mM D-luciferin potassium salt. Bioluminescence was measured at room temperature for 10 seconds per well, using a Centro XS³ LB 960 plate-reading luminometer (Berthold Technologies, Germany).

Regarding the irradiation intensity dependence experiments shown in Figure 9 and Figure 11, COS-7 cells were seeded at 10,000 cells/well or 15,000 cells/well in a 96-well transparent, flat-bottom, black-walled microplate, and were then cultured at 37°C under conditions of 5% CO₂ for 24 hours. The cells were transfected with cDNA encoding scPA-Cre and a loxP-stop-loxP Fluc reporter at a ratio of 1 : 10, using X-tremeGENE 9 reagent at 37°C. The total amount of the DNA used was set to 0.05 µg/well. The cells were transfected with cDNA encoding scPA-Flp and a loxP-stop-loxP Fluc reporter at a ratio of 1 : 10, using Lipofectamine 3000 reagent at 37°C. The total amount of the DNA used was set to 0.1 µg/well. Twenty-four hours after the transfection, in the dark, the cells were irradiated with a blue light (1.0 W/m⁻²) for 30 seconds, 3 minutes, 30 minutes, and 1 hour, and thereafter, the cells were cultured in the dark, or were irradiated with a blue light irradiation (1.0 W/m⁻²) for 24 hours. Immediately before the luciferase assay, the medium was exchanged with an HBSS medium containing 0.2 mM D-luciferin potassium salt. Bioluminescence was measured at room temperature for 10 seconds per well, using a Centro XS³ LB 960 plate-reading luminometer (Berthold Technologies, Germany).

Regarding the recombination cascade experiment shown in Figure 15, COS-7 cells were seeded at 15,000 cells/well in a 96-well transparent, flat-bottom, black-walled microplate, and were then cultured at 37°C under conditions of 5% CO₂ for 24 hours. The cells were transfected with cDNA encoding iCre or pcDNA3.1 (+) empty vector, cDNA encoding scPA-Flp, and a loxP-stop-loxP Fluc reporter at a ratio of 1 : 1 : 9, using Lipofectamine 3000 reagent at 37°C. The total amount of the DNA used was set to 0.1 µg/well. The cells were transfected with cDNA encoding PA-Flp or scPA-Flp and a loxP-stop-loxP Fluc reporter at a ratio of 1 : 10, using Lipofectamine 3000 reagent at 37°C. The total amount of the DNA used was set to 0.1 µg/well. Twenty-four hours after the transfection, the cells were cultured in the dark or under blue light irradiation (1.0 W/m⁻²) for 24 hours. Immediately before the luciferase assay, the medium was exchanged with an HBSS medium containing 0.2 mM D-luciferin potassium salt. Bioluminescence was measured at room temperature for 10 seconds per well, using a Centro XS³ LB 960 plate-reading luminometer.

### 1-8. In vivo experiments using mice

Experiments using mice were conducted in accordance with the Guide for the Care and Use of Laboratory Animals. Six-week-old female Rosa26-CreER^{T2} mice (CreER^{T2} mice) (kindly provided by Dr. Tyler Jacks, Massachusetts Institute of Technology) were used in all experiments. Hair on the abdominal surface was removed with depilatory cream. The depilated mice were allowed to rest in their cages for at least 12 hours. Six hours before HTV (hydrodynamic tail vein) injection of plasmid cDNA, in order to activate CreER^{T2} recombinase, the mice were intraperitoneally injected with 100 µg/body weight (g) of tamoxifen (Sigma). Subsequently, HTV injection of the cDNA encoding pcDNA3.1-loxP-stop-loxP-scPA-Flp and the cDNA encoding pcDNA3.1-FRT stop-FRT-Akaluc-mKate2 into the mice was performed at a ratio of 1 : 1, using TransIT-QR Hydrodynamic Delivery Solution (Mirus Bio LLC) according to the attached instructions for use. The total amount of the DNA injected was set to 40 µg/mouse. The total volume of the solution injected was set to 0.1 mL/mouse. After the DNA injection, the mice were placed in a cage and were reared in the dark for 6 hours. Thereafter, the mice were irradiated with an LED light source (470 ± 20 nm; 132 W/m⁻²) for 18 hours. Fluorescence imaging of the mice was performed 24 hours after the DNA injection. The livers of the DNA-injected mice were isolated using standard surgical techniques. The isolated livers were immersed in a PBS solution. Subsequently, the fluorescence images of the isolated livers were captured using an all-in-one fluorescence microscope BZ-X710 (KEYENCE).

### 1-9. Imaging of living cells

A glass-bottom dish (Cat. #D11530H, Matsunami) was treated with 25 mg/mL human fibronectin (BD Biosciences) at room temperature for 10 minutes, and was then washed twice with 2 mL of Milli-Q water. In order to perform cell migration assays using confocal imaging, NIH3T3 cells were seeded onto a 35-mm glass-bottom dish (glass area = φ14 mm) at 2,000 cells/dish, and were then cultured at 37°C under conditions of 5% CO₂ for 24 hours. The cells were transfected with Lifeact mCherry and cDNA encoding scPA-Vav or 2PscPA-Vav, using Lipofectamine 3000 reagent, at 37°C. The total amount of the transfected DNA was set to 0.1 µg/dish. Twenty-four hours after the transfection, the medium was exchanged with Leibovitz's L-15 phenol red free medium supplemented with 5% FBS and 2 mM GlutaMAX. Imaging was performed using a x63/1.05 numerical aperture (NA) silicone oil immersion objective lens (Carl Zeiss) on the stage of an LSM confocal laser scanning microscope (Carl Zeiss) equipped with a heated stage adapter (Tokai Hit). The fluorescence images of mCherry were acquired using a multiline HeNe laser (543 nm). One-photon blue light irradiation was performed with a 1 mW multiline argon laser (488 nm). Two-photon excitation of mTagBFP2 was performed with a 25 mW multiline Chameleon laser (808 nm).

### 1-10. Quantitative analysis of cell area extension

The fluorescence images of Lifeact-mCherry and, NIH3T3 cells expressing scPA-Vav or 2PscPA-Vav, were acquired 15 minutes before light irradiation (dark), immediately before light irradiation (before), and 15 minutes after light irradiation (after). The acquired fluorescence images were converted to binary data, based on fluorescence intensity of mCherry, using Image-J software. The obtained data were used to calculate the percentage of cell area expansion percentage (%) between the (dark) and (before) image, or the (before) and (after) image.

### 1-11. Reproducibility and statistical analysis

No power tests were performed in the cell culture experiments and the mouse experiments. There were neither samples nor animals that were excluded from the analysis. Randomization methos were performed in the cell culture experiments and the mouse experiments . No blinding was performed in the cell culture experiments or the mouse experiments.

### 2. Results

### 2-1. Development of scMagnet

Based on the split Cas9 endonuclease and the Magnet system (Kawano et al., Nat. Commun. 6:6256 (2015)), the present inventors have developed a two-molecule-type photoactivatable Cas9 (PA-Cas9) (Nihongaki et al., Nat. Biotechnol. 33:755-760 (2015)) that is useful for performing optogenetic genome editing (see Figure 2a, Figure 3, and Figure 4). First, the efficiency of light induction of conventional PA-Cas9 activity was measured using the tracking of indels by decomposition (TIDE) assay (Brinkman et al., Nucleic Acids Res. 42: e168 2014). Under blue light irradiation, HEK293T cells expressing PA-Cas9 targeting the human VEGFA gene showed a high indel mutation introduction percentage of over 25%, which was equivalent to that of cells expressing full-length Cas9 (Figure 1b). In addition, PA-Cas9 highly efficiently introduced indel mutations also into the human DNMT1 gene and EMX1 gene in HEK293T cells in a light-dependent manner (Figure 5).

On the other hand, HeLa cells expressing PA-Cas9 targeting the human VEGFA gene showed a very low indel mutation introduction percentage of less than 1% under both conditions, namely, in-the-dark and under blue light irradiation (Figure 2d). Furthermore, as in the case of targeting the human VEGFA gene, HeLa cells expressing PA-Cas9 targeting the human DNMT1 gene and EMX1 gene also showed a low indel mutation introduction percentage, which was almost the same level as that of cells expressing the pcDNA3.1 empty vector (Figure 5). In general, since the expression level of a foreign protein is known to be lower in HeLa cells than in HEK293T cells (Rio et al., Science 227:23-28 (1985)), the present inventors have assumed that the low light induction efficiency of PA-Cas9 in HeLa cells would be caused by the low expression level of PA-Cas9 in the cells.

In order to solve the problem of two-molecule-type photoswitching systems such as the conventional Magnet system (for example, the conventional Magnet system is composed of two molecules, namely, pMag and nMag), namely, the problem that the light induction efficiency depends on the concentration of the Magnet protein in the cell, the present inventors have conceived of linking pMag and nMag in series, as shown in Figure 2c. This type of two Magnet protein molecules linked in series is referred to as scMagnet (single-chain-based Magnet).

Each split Cas9 fragment was allowed to bind to pMag and nMag, respectively, and they were then linked to each other in tandem to create scPA-Cas9 (single-chain-based photoactivatable Cas9) (Figure 2c). The light induction efficiency of the genome editing thereof (i.e., introduction of indel mutations into the target genomic region) was measured. As a result, as expected, in HeLa cells expressing scPA-Cas9 targeting the human VEGFA gene, a high indel mutation of over 10% was introduced into the VEGFA gene region (Figure 2d). In contrast, in the dark, a low indel frequency of approximately 1% to 4%, which was equivalent to that of the pcDNA3.1 empty vector, was shown. From these results, it was demonstrated that the light induction efficiency of scPA-Cas9 activity in HeLa cells is approximately 18.1-fold higher than the light induction percentage of PA-Cas9 activity, suggesting a significant improvement. The features of scPA-Cas9, by which it shows high genome editing efficiency under blue light irradiation and hardly conducts genome editing in the dark, are shown also in the case of targeting other genes (human DNMT1 gene and EMX1 gene) in HeLa cells (Figure 6).

The aforementioned results demonstrate that scMagnet functions as a one-molecule-type photoswitching system, which is characterized in that it has low leakage activity in the dark (i.e., binding activity in the dark) and an extremely high rebinding induction percentage upon blue light irradiation.

### 2-2. Verification of in vitro scMagnet action

### 2-2-1. Versatility of scMagnet

Next, whether the scMagnet system can be applied to the optogenetic rebinding of other split proteins other than split Cas9 was studied. Previously, the present inventors have developed photoactivatable Cre recombinase (PA-Cre) (Kawano et al., Nat. Chem. Biol. 12: 1059-1064 (2016)) and photoactivatable Flp recombinase (PA-Cre) (Jung et al., Nat. Commun. 10: 314 (2019)) based on the Magnet system. Thus, in order to study the versatility of the scMagnet system, the present inventors have constructed single-stranded photoactivatable Cre recombinase and single-stranded photoactivatable Flp recombinase based on the scMagnet, and have then studied their activity (Figures 7a and c, Figure 8, and Figure 10). First, the present inventors have performed a luciferase reporter assay in COS-7 cells, so that the light induction efficiency of scPA-Cre or scPA-Flp was compared with the light induction efficiency of A-Cre or PA-Flp, respectively. As a result, as with PA-Cre, scPA-Cre exhibited highly efficient light-inducible recombination activity (achieved 122-fold) under continuous under-light irradiation, under short-term pulse irradiation, and under-light irradiation of different intensities, and exhibited no leakage activity in the dark (Figure 9). Moreover, as with PA-Flp, scPA-Flp also exhibited highly efficient light-inducible recombination activity (achieved 122-fold) and no leakage activity in the dark (Figure 11). From these results, it was demonstrated that the scMagnet can induce the optogenetic rebinding of not only split Cas9 endonuclease but also other split proteins, including split Cre recombinase and split Flp recombinase.

Subsequently, the influence of suppression of the expression levels of scPA-Cre and PA-Cre in COS-7 cells upon the recombination efficiency of scPA-Cre and PA-Cre was studied. The transfection amounts of plasmid DNA encoding scPA-Cre and plasmid DNA encoding PA-Cre were reduced from 9.1% (the experimental conditions for the data shown in Figure 9) to 4.8% of the total transfection amount. In this case, even in a case where the expression level was reduced, scPA-Cre exhibited higher light-inducible recombination activity than PA-Cre under blue light irradiation (Figure 7b). Similar results were observed when split Flp recombinase was used (Figure 7d). Furthermore, similar results were obtained, even when NIH3T3 cells, in which the expression levels of exogenous gene proteins were lower than in COS-7 cells, were used (Figure 12).

These results demonstrate that the scMagnet system can efficiently control the optogenetic rebinding of split proteins even in cells in which the expression levels of exogenous gene proteins are low.

### 2-2-2. Influence of linker length or amino acid deletion, in linking portion of pMag and nMag, on activity

The influence of the length of a linker that links pMag and nMag on the light induction efficiency by the scMagnet system was studied. scPA-Cre, in which pMag is linked to nMag with a flexible peptide linker (a linker consisting of 0 to 20 amino acid residues), was produced (Figure 13a).. The light induction efficiency of recombination activity of scPA-Cre with no inserted linkers was compared with that of scPA-Cre with an inserted flexible linker (Figure 13a). The term "iCre" is a control in which a nuclear localization signal is linked to full-length Cre and which does not respond to a blue light. The term "reporter only" is a control in which neither scPA-Cre nor iCre is used and which consists only of the bioluminescence reporter. As a result of the experiments, regardless of linker length, recombination activity was observed in all scPA-Cre with inserted flexible linkers, with high light induction efficiency that was equivalent to the light induction efficiency of scPA-Cre with no inserted linkers (Figure 13a). These features of the scMagnet system were also confirmed in the case of genome editing using Cas9 (Figure 14).

Subsequently, it was found that the leakage activity of scPA-Cre (in the case of no linkers) was further suppressed by deleting several amino acid residues at the C-terminus of pMag (Figure 13b). It is to be noted that since deleting amino acid residues at the C-terminal side of pMag tends to reduce the light induction efficiency of recombination, the number of amino acids deleted is not particularly limited, but approximately 6 is preferable.

Furthermore, the influence of deleting several amino acid residues at the N-terminus of nMag on the activity was studied. The enzyme activity of scPA-Cre, which was formed by directly linking the C-terminus of pMag and the N-terminus of nMag to form one-molecule-type scPA-Cre, and the enzyme activity of scPA-Cre mutants (Δ1 to Δ19) introduced with mutants in which the N-terminus of the nMag was deleted one amino acid at a time, were measured in the dark and under blue light irradiation (Figure 15). The amino acids around the linking portion of pMag and nMag are shown in Figure 15. The term "iCre" is a control in which a nuclear localization signal is linked to full-length Cre and which does not respond to a blue light. The term "reporter only" is a control in which neither scPA-Cre nor iCre is used and which consists only of the bioluminescence reporter.

As a result of the measurements, it was found that each scMagnet mutant, in which one amino acid from positions 1 to 19 of nMag was deleted, exhibited low leakage activity in the dark and retained high responsiveness to a blue light.

The Cre recombination activity was studied, when amino acids on the N-terminal side of nMag were further deleted stepwise (Figure 16). The amino acid sequence and domain structure of nMag are shown in Figure 16A. An scPA-Cre mutant was constructed by introducing an nMag mutant deleting amino acids that were less than the amino acid number indicated by the symbol Δ in the figure, and the Cre recombination activity thereof was evaluated (Figure 16B). In addition, an scPA-Cre mutant (pMag+nMag-Latch-NCap-Hinge) was also constructed by introducing an nMag mutant having only the Latch-NCap-Hinge, and the Cre recombination activity thereof was evaluated. The enzyme activity of single-chain photoactivatable Cre recombinase (scPA-Cre, pMag+nMag), in which the N-terminal fragment of split-Cre and the C-terminal fragment of split-Cre were introduced into both termini of scMag, respectively, the enzyme activity of the scPA-Cre mutant (Δ35, Δ56, Δ71, or Δ119), in which the N-terminus of nMag was largely deleted, the enzyme activity of an scPA-Cre mutant (Latch-NCap-Hinge), in which nMag was introduced into only the N-terminal region, and the enzyme activity of scPA-Cre mutant (ΔnMag), in which nMag was not introduced but only pMag was introduced, were measured in the dark and under blue light irradiation. The results are shown in Figure 16B. The term "iCre" is a control in which a nuclear localization signal is linked to full-length Cre and which does not respond to a blue light. The term "reporter only" is a control in which neither scPA-Cre nor iCre is used and which consists only of the bioluminescence reporter.

As a result of the measurements, it was found that the leakage activities of the scMagnet mutants in the dark were low even when the amino acids up to position 35 of nMag were deleted, but that the leakage activities of the scMagnet mutants in the dark were significantly increased when the amino acids up to position 56 of nMag were deleted. From these results, it is considered that the leakage activity of scPA-Cre introduced with nMag mutants, in which the amino acid residues on the N-terminal side were deleted, changes from "low" to "high" by deleting any of the amino acid residues at positions 36 to 55 from the N-terminal amino acid residues of nMag.

Subsequently, the enzyme activity of scPA-Cre, and the Cre recombination activities of scPA-Cre mutants introduced with mutants in which the C-terminus of pMag and the N-terminus of nMag were each partially deleted, were measured in the dark and under blue light irradiation (Figure 17). The term "iCre" is a control in which a nuclear localization signal is linked to full-length Cre and which does not respond to a blue light. The term "reporter only" is a control in which neither scPA-Cre nor iCre is used and which consists only of the bioluminescence reporter.

As is found from Figure 17, by linking the pMag mutant with a deletion of the amino acid residues on the C-terminal side and the nMag mutant with a deletion of the amino acid residues on the N-terminal side, there could be obtained an scMagnet mutant that exhibited low leakage activity in the dark and retained high responsiveness (recombination activity) under blue light irradiation.

### 2-3. Verification of in vivo scMagnet action

Next, the in vivo action of the scMagnet system was verified using scPA-Flp. First, a recombination cascade system based on light-inducible recombination of scPA-Flp, whose expression is induced by Cre recombination, was constructed (Figure 18a). First, using this system, it was confirmed in vitro that scPA-Flp recombination is induced with high efficiency in COS-7 cells, not only by blue light irradiation but also by Cre expression (Figure 18b). Subsequently, in order to operate the present recombination cascade system in vivo, experiments were performed using the Rosa26-activable CreER^{T2} mouse models (Ventura et al., Nature 445: 661-665 (2007)), which unevenly express tamoxifen-inducible Cre recombinase. Using the hydrodynamic tail vein injection method (Liu et al., Gene Ther. 6: 1258-1266 (1999)), plasmid DNA encoding a floxed-stop scPA-Flp and plasmid DNA encoding an FRT-floxed-stop mKate2 reporter were introduced into mouse livers (Figure 19a and b, and Figure 20). The mice injected with the DNA were reared in the dark or under blue light irradiation for 18 hours. When the mice were reared under blue light irradiation, mKate2 fluorescence was detected in the liver of mice injected with tamoxifen (Figure 19c and Figure 20). In contrast, no fluorescence was detected in the liver of mice reared in the dark (Figure 19c and Figure 20). Regarding fluorescence, the fluorescence of mKate2 was not detected in mice without tamoxifen injection, which were reared in the dark or under blue light irradiation. These results demonstrate that scPA-Flp functions well in vivo in the presence of Cre and under blue light irradiation (Figure 19c and Figure 20).

From the aforementioned results, it was demonstrated that scMagnet functions in vivo, even by irradiation with a blue light that is non-invasive to the living body.

### 2-4. Studies regarding reversibility of function of scMagnet

Using the Rho-family guanine exchange factor (GEF) Vav2 protein, which is necessary for cell expansion, whether or not scMagnet can reversibly function in a blue light irradiation-dependent manner was studied (Figure 21).

First, scMagnet utilizing photoactivatable Vav2 (referred to as scPA-Vav) was constructed (Figure 22a and b). Using Lifeact-mCherry as a fluorescent biosensor to detect actin polymerization, the activity of scPA-Vav before and after the blue light irradiation was evaluated by a cell area expansion assay (Figure 23).

When NIH3T3 cells expressing scPA-Vav were maintained in the dark for 15 minutes, there was almost no change in the cell area. However, when the NIH3T3 cells were irradiated with a blue light for 15 minutes, significant cell area expansion was induced (Figure 22c and d, and Figure 24). On the other hand, in the presence of EHop-016, which inhibits the binding between Vav2 and Racl1, the cell area expansion was not observed as in the case of maintaining the cells in the absence of EHop-016 in the dark (Figure 22e and f, and Figure 24). These results demonstrate that scPA-Vav does not cause leak activity in the dark and effectively activates Vav2 signaling in a blue light-dependent manner.

Subsequently, in order to examine whether scPA-Vav functions reversibly, scPA-Vav-expressing cells were irradiated with a blue light and were then maintained in the dark. As a result, scPA-Vav induced expansion of the cell area by blue light irradiation for 15 minutes, but the expanded cell area disappeared when the cells were maintained in the dark for 15 minutes (Figure 22g). These results demonstrate that scMagnet can reversibly control the binding and dissociation of split proteins.

### 2-5. Control of scMagnet system by two-photon excitation

When the scMagnet system is allowed to function in biological tissues, if two-photon excitation using a long-wavelength near-infrared light is utilized, it is considered that scMagnet can be efficiently activated in a deeper portion in the biological tissues than one-photon excitation. Moreover, since a two-photon excitation microscope has higher spatiotemporal resolution than a one-photon excitation microscope, it has been utilized to more precisely control protein activity. Thus, activation of scMagnet was attempted using two-photon excitation.

The activation efficiency of flavin-binding photoswitching proteins, including the Magnet system, by two-photon excitation is extremely low because the two-photon absorption cross-sectional area of the flavin cofactor is small (Kinjo et al., Nat. Methods 16 :1029-1036 (2019)), and therefore, there have been no previous examples of successful control of the optogenetic rebinding of split proteins using the two-photon excitation. Thus, it was conceived that activation by two-photon excitation (Kinjo et al., Nat. Methods 16: 1029-1036 (2019); Kinjo et al., ACS Chem. Biol. 15: 2848-2853 (2020)), in which the principle of fluorescence resonance energy transfer (FRET) is utilized, would be applied to the scMagnet system. The scMagnet system enables efficient optogenetic control by linking pMag and nMag with a flexible linker (see Figure 2c, Figure 7a and b, etc.). Thus, an attempt was made to insert the fluorescent protein mTagBFP2 into the linker portion linking pMag and nMag, so that the resultant was allowed to function as a donor of FERT (Figure 25).

Since the distance between the two chromophores significantly affects FRET efficiency, the distance between mTagBFP2 and the Magnet system (pMag and nMag) is desirably shorter. Based on the structural models of mTagBFP2 (a.a. 1-237) and the Magnet system, it was determined that the N-terminal domain (a.a. 1-6) and C-terminal domain (a.a. 229-237) of mTagBFP2 could be deleted. Thus, using a scMagnet in which ΔmTagBFP2 (a.a. 7-228) was inserted between pMag and nMag (2PscMagnet), a two-photon excitation-type Vav2 (2PscPA-Vav) was constructed (Figure 25b and c).

First, in order to demonstrate the photoresponsiveness of 2PscPA-Vav, cell expansion assays were performed using one-photon excitation with a blue light of 488 nm. NIH3T3 cells expressing 2PscPA-Vav exhibited highly efficient light-inducible cell area expansion (Figure 25d and Figure 26).

Subsequently, the photoresponsiveness of 2PscPA-Vav was examined using a two-photon excitation microscope. When two-photon excitation was performed using a near-infrared light of 808 nm, the NIH3T3 cells expressing 2PscPA-Vav showed significant cell area expansion, compared to the case of maintaining the cells in the dark (Figure 25e and f, and Figure 26). The efficiency of cell area expansion by two-photon excitation was comparable to that by one-photon excitation. On the other hand, even if the NIH3T3 cells expressing scPA-Vav were subjected to two-photon excitation (for 15 minutes), cell area expansion was not induced (Figure 25g and Figure 27).

From the aforementioned results, it was demonstrated that 2PscMagnetis activated by two-photon excitation, but that scMagnet is not activated by two-photon excitation.

### Industrial Applicability

According to the present invention, provided is a photoswitching protein that can precisely control the binding and dissociation of proteins by turning a light ON/OFF in light irradiation. The photoswitching protein of the present invention can efficiently control the binding and dissociation of various proteins in cells in vitro and in vivo. Therefore, it is expected that the photoswitching protein of the present invention will be utilized in the life science research field, in the medical field for the purpose of treating diseases, etc., and in other fields.

## Claims

1. A protein, in which the C-terminus of the following protein (a) is directly or indirectly linked to the N-terminus of the following protein (b):
(a) a protein comprising an amino acid sequence having a sequence identity of 80% or more to an amino acid sequence comprising a deletion of an X number of consecutive amino acid residues from the C-terminal residue to the N-terminal side of the amino acid sequence as set forth in SEQ ID No: 1 or SEQ ID No: 36, wherein X represents an integer of 0 or greater and 6 or smaller; and
(b) a protein comprising an amino acid sequence having a sequence identity of 80% or more to an amino acid sequence comprising a deletion of a Y number of consecutive amino acid residues from the N-terminal residue to the C-terminal side of the amino acid sequence as set forth in SEQ ID No: 1 or SEQ ID No: 36, wherein Y represents an integer of 0 or greater and 55 or smaller,
provided that when X of the protein (a) is 0, Y of the protein (b) is an integer of 1 or greater and 55 or smaller, and when Y of the protein (b) is 0, X of the protein (a) is an integer of 1 or greater and 6 or smaller.

2. The protein according to claim 1, wherein, in the amino acid sequence of the protein (a), when the amino acids that correspond to positions 52 and 55 on the amino acid sequence as set forth in SEQ ID No: 1 are substituted with arginine, or when the amino acids that correspond to positions 16 and 19 on the amino acid sequence as set forth in SEQ ID No: 36 are substituted with arginine, in the amino acid sequence of the protein (b), the amino acid that corresponds to position 52 on the amino acid sequence as set forth in SEQ ID No: 1 is substituted with aspartic acid and the amino acid that corresponds to position 55 thereon is substituted with glycine, or the amino acid that corresponds to position 16 on the amino acid sequence as set forth in SEQ ID No: 36 is substituted with aspartic acid and the amino acid that corresponds to position 19 thereon is substituted with glycine.

3. The protein according to claim 1, wherein the C-terminus of the protein (a) is linked to the N-terminus of the protein (b) via a polypeptide.

4. The protein according to claim 3, wherein the polypeptide is a fluorescent protein or a bioluminescent protein.

5. The protein according to claim 4, wherein the fluorescent protein is any one of BFP, TagBFP, mTagBFP2, mBlueberry2, mCerulean, mTurquoise, ECFP, TagCFP, Rosmarius, meleCFP, mTFP1, KCY, meffCFP, GFP, YFP, Venus, mCherry, and iRFP.

6. The protein according to claim 4, wherein the bioluminescent protein is any one of NanoLu, Gaussia luciferase, Renilla luciferase, and firefly luciferase.

7. A nucleic acid encoding the protein according to any one of claim 1 to claim 6.

8. A method for controlling the distance between two proteins,
in which, in the protein according to any one of claim 1 to claim 6, the method comprises a step of binding the protein (a) to one of the two proteins, a step of binding the protein (b) to the other of the two proteins, and irradiating the protein according to any one of claim 1 to claim 6 with a light.

9. The method according to claim 8, wherein the two proteins are interacting proteins.
